# EUROPEAN PATENT APPLICATION

(11) **EP 3 932 411 A1**
(43) Date of publication of application: **05.01.2022**
(21) Application number: 20183323.3
(22) Date of filing: 30.06.2020
(51) Int. Cl.: A61K 31/737, A61P 31/16

(54) **ANTIVIRAL PHARMACEUTICAL COMPOSITION**

(71) Applicant: GSK Consumer Healthcare SARL, 1260 Nyon (CH)
(72) Inventor: Lombardi, Maria Stella, 1260 Nyon (CH); Bulsara, Pallav, Warren, New Jersey 07059 (US)
(74) Representative: Gundel, Isabelle

(57) **Abstract**

Pharmaceutical compositions are described comprising fucoidan for use in a method for the treatment or the prevention, by intranasal administration, of an infection with rhinovirus or respiratory syncytial virus in a human. Particularly, the viral infection is HRV-C15, HRV-A16, or HRV-B14 infection or RSV type A infection. There are also described pharmaceutical compositions comprising fucoidan for use in a method for prevention of rhinovirus spread or respiratory syncytial virus spread in a human population, wherein subjects of the population are tested for infection with HRV-C15, HRV-A16, or HRV-B14 or RSV type A and infected subjects are treated with intranasal administration of the pharmaceutical composition.

## Description

### FIELD OF THE INVENTION

The present invention relates to pharmaceutical compositions for use in methods of treatment and prevention of infection of the respiratory tract by rhinovirus or respiratory syncytial virus, particularly human rhinovirus strain HRV-C15, HRV-A16, or HRV-B14, or respiratory syncytial virus A. The present invention further relates to the control of the spread of infection with rhinovirus or respiratory syncytial virus, particularly human rhinovirus strain HRV-C15, HRV-A16, or HRV-B14, or respiratory syncytial virus A.

### BACKGROUND TO THE INVENTION

Acute respiratory viral infection (ARVI) is an acute infection of the respiratory tract, present in the form of catarrhal inflammation of the upper respiratory airway, and progressing with fever, runny nose, sneezing, cough and sore throat. The illness disturbs the general health to different extents. It may end in spontaneous recovery but may also cause severe illness and complications even leading to death of the patient. ARVIs can be provoked by more than 200 species of respiratory viruses. Most common are rhinovirus-provoked ARVIs: more than 100 serotypes of rhinoviruses cause approximately 50% of all ARVIs.

Rhinoviruses belong to the family of Picornaviridae. They are non-enveloped and have a single strand, positive sense RNA genome. Rhinoviruses (RV) are classified into groups A, B and C according to their phylogenetic sequence and distinct genomic features. Human rhinoviruses (HRV) of all three types frequently cause ARVIs, but strains of rhinovirus of group A (HRV-A) and strains of rhinovirus of group C (HRV-C) are more likely than strains of rhinovirus of group B (HRV-B) to be associated with severe illness and complications as described above. In particular, RV or RV coinfections were detected in ∼90% of kids hospitalized with acute asthma episodes, of which HRV-C was the primary agent, presenting most frequently (59.4%) in patients with the severest symptoms (Bizzintino J, 2011. Association between human rhinovirus C and severity of acute asthma in children. Eur Respir J37:1037-1042. doi:10.1183/09031936.00092410*).* There is no pharmaceutical composition commercially available for the treatment or prevention of rhinovirus infection. Rupintrivir is a drug that is effective against rhinoviruses *in vitro but* was never marketed as a medicine for humans because it was not effective *in vivo.* The three groups of rhinoviruses infect a host cell by binding to different plasma membrane glycoproteins which leads to receptor-mediated endocytosis of the virus into the host cell.

Infection with respiratory syncytial virus (RSV) can also cause ARVI and cause severe illness and complications, such as bronchiolitis, which accounts for a high proportion of hospitalisations of children and infants. RSV belong to the family of Pneumoviridae. They are enveloped viruses with a linear negative sense single strand RNA genome. RSV are grouped into subgroups A and B on the basis of their affinity to specific antibodies, wherein subtype A is responsible for more severe symptoms. It has not yet been clarified which cellular receptor is the primary point of entry for RSV into human host cell. Binding of RSV to various host cell receptors has been observed in different cell models. RSV infection is mostly treated symptomatically, and in severe cases, receptor antibodies may be administered. However, antibodies are costly, used as prophylactic treatment only for high risk infants and are not spared from side effects. Alternatively, ribavirin, the only antiviral drug approved by the FDA for treatment of RSV, can be administered.

Modes of infection and reproduction differ greatly between the different families of respiratory viruses. Current antiviral drugs for ARVI treatment target key molecules in binding, endocytosis, fusion (uncoating), DNA or RNA synthesis and replication, assembly and release of the viruses. Consequently, they are effective against a specific family of viruses that uses those target molecules. Even within one virus family and species, effectiveness may vary due to different mechanisms, genotypes and expression of the target proteins. Due to innate or acquired mutation, different strains of viruses may be less susceptible or even resistant to drug treatment. Also, patients do often not have the possibility to get tested for viral infection, so that it is not always possible to select an appropriate antiviral treatment. This is because the most tests for ARVI today require taking of specimens in a clinical setting and analysis of the obtained specimen in a laboratory. Testing in clinical setting, laboratory capacity and testing equipment are limited. Currently available antiviral drugs are generally administered systemically, such as per oral or intravenous administration. This results in systemic concentrations of the drug that may lead to side effects. Therefore, local treatment for ARVI that effectively stops and kills the virus at the initial site of infection, the respiratory mucosa, is highly desirable. There is also a need for pharmaceutical compositions that can be used to prevent ARVI infection in humans and are easy to use, and with a low risk of side-effects.

Fucoidan, a marine polysaccharide, has been tested against various respiratory virus families, genus, species and strains with mixed results.

WO2009/027057 teaches antiviral compositions comprising a sulphated polysaccharide and discloses that carrageenan and fucoidan inhibit the parainfluenza virus 3 mediated cell death in HNep cells when the cells were inoculated in the presence of the polysaccharides, wherein carrageenan is more effective than fucoidan (example 13). Similar results are achieved in the same experiment after inoculation with influenza virus H3N2, wherein iota-carrageenan has the best protective effect at most concentrations (example 14). The disclosure further teaches that pretreatment of the virus with fucoidan cannot reduce plaque formation of avian influenza virus H5N1 in MDCK cells (example 15).

WO2011/100805 teaches methods for inhibiting a virus of the orthomyxoviridae family comprising contacting the virus or a cell infected with the virus with an effective amount of a formulation comprising a sulfated polysaccharide having an average molecular weight of 4,000 Daltons or greater. Sulfated polysaccharide formulations reduce the cytopathic effect (CPE) of Influenza A type H1N1, strain California/07/2009 in MDCK cells, but are not effective in the same experiment against influenza A type H3N2, strain Brisbane/10/2007.

Wang et. al. (Wang, W., Wu, J., Zhang, X. et al. Inhibition of Influenza A Virus Infection by Fucoidan Targeting Viral Neuraminidase and Cellular EGFR Pathway. Sci Rep 7, 40760 (2017). DOI: 10.1038/srep40760) found that fucoidan from *Kjellmaniella crassifolia* reduces CPE and plaque formation in MDCK cells after infection with different H1N1 strains (PR8, Cal09 and TX09) as well as the H3N2 strain Minnesota. It was further found in a surface plasmon resonance (SPR) assay that fucoidan binds to the Neuraminidase (NA) protein of the subtypes H1N1 (Cal09) and H3N2 (Minnesota). Authors suggest that fucoidan may inhibit the entry and release process of influenza A virus through direct binding to influenza A NA.

Fucoidan is thus only effective in some virus families, and the efficacy against a certain virus cannot be predicted. Even within one family, the response varies greatly between different species and even strains. Whilst a mechanism was proposed for the effect of fucoidan in influenza A virus, fucoidan compositions have been tested in different species and strains of influenza A with mixed results. Furthermore, the proposed mechanism is limited to influenza A viruses as it is dependent on a target molecule specific to that family.

### SUMMARY OF THE INVENTION

In a first aspect, the invention is directed to a pharmaceutical composition comprising fucoidan for use in a method for the treatment or the prevention, by intranasal administration, of an infection with rhinovirus or respiratory syncytial virus in a human. The present inventors found that the composition of the present invention, administered to human nasal epithelium, reduces the replication of rhinovirus strains HRV-C15, HRV-A16 and HRV-B14. As the composition of the present invention is effective against rhinoviruses of all three groups, it is believed that composition is effective broadly against all strains of rhinovirus. Furthermore, the present inventors also found that the composition of the present invention, administered to human nasal epithelium, reduces the replication of respiratory syncytial virus type A, and is believe it to be effective against all types of RSV.

The pharmaceutical compositions of the present invention are therefore effective in the treatment of infection with rhinovirus and in the treatment of infection with respiratory syncytial virus infection in a human. The pharmaceutical compositions achieve a reduction of the viral load, or the viral titre, of the subject. Both parameters can be determined by measures as known in the art, for example quantitative real time polymerase chain reaction (RT-PCR). In one embodiment, the treatment reduces the nasal viral load of a subject. In one embodiment, the nasal viral load is determined by RT-PCR, performed on a nasal swab specimen from the subject.

The invention comprises a method for treating infection with rhinovirus or respiratory syncytial virus comprising a method of detecting viral RNA of rhinovirus or respiratory syncytial virus from a specimen obtained from the subject and, where viral RNA is detected, a step of treatment as described herein. The method of detecting viral RNA of rhinovirus or respiratory syncytial virus from a specimen obtained from the subject may use a CRISPR diagnostic technique (for instance using the DECTECTR^{™} method using Cas12 (Chen et al., Science 360, 436-439 (2018), or using the SHERLOCK^{™} method using Cas13 (Gootenberg et al., Science 356: 438-442 (2017)). Alternatively, the method of detecting viral RNA from a specimen obtained from the subject may use reverse-transcriptase polymerase-chain-reaction (RT-PCR) assays or other diagnostic methods that are known in the art.

The pharmaceutical compositions of the present invention are also suitable for the prevention of infection with rhinovirus or respiratory syncytial virus in humans. For prevention of rhinovirus infection or respiratory syncytial virus infection, the pharmaceutical compositions of the invention are administered intranasally to subjects that have not been tested for rhinovirus infection or respiratory syncytial virus infection, or to subjects that have been tested for rhinovirus infection or respiratory syncytial virus infection and the result were negative. The pharmaceutical compositions are effective and useful in prevention of rhinovirus and respiratory syncytial virus infection as, if applied preventively, hinder virus from infecting cells of the nasal epithelia, are not harmful to the nasal epithelia and are conveniently applied intranasally. As fucoidan is authorised as a food supplement in doses up to 500 mg per day, the compositions of the present invention are considered safe for oral intake if swallowed and have a low risk of adverse events.

In one embodiment, the infection is HRV-C15 infection, or HRV-A16 infection, or HRV-B14 infection. In another embodiment, the infection is respiratory syncytial virus type A infection. As found by the present inventors, the compositions of the present invention are effective in reducing the viral replication of HRV-C15, HRV-A16, HRV-B14 and RSV type A in nasal epithelia. As an effect has been established for rhinovirus strains of all three groups, a similar effect is expected in other strains of rhinovirus.

According to the invention, the treatment comprises intranasal administration of pharmaceutical compositions of the present invention. This comprises nasal administration of solutions, gels, creams, powders or foams. The administration can be by pipetting, dropping or spraying of the pharmaceutical composition into the nostrils, by nebulising or atomising of the composition and inhalation of the nebulised or atomised composition or by applying of the composition to the nasal epithelia.

In one embodiment, the composition is a nasal spray, and the intranasal administration is thus the administration of a nasal spray. Administration of a nasal spray comprises inserting a nozzle into a nostril, activating a spray mechanism that expels and atomises the composition through the nozzle and into the nostril, optionally concerted inhalation and replication of the same steps with the second nostril. The resulting droplets or particles are then deposited onto the nasal epithelia. Naturally occurring ciliary beating transports the compositions to the adjacent epithelia of the nasopharynx, oropharynx and laryngopharynx. Nasal spray administration therefore is useful to bring the composition of the present invention to the initial point of infection for rhinoviruses and respiratory syncytial viruses (Jospeh B. Domachowske and Helene F. Rosenberg, Clinical Microbiology Reviews, 1999, p. 298-309), the epithelial cells of the upper respiratory tract. This is possible without intervention of a health care professional and without clinical intervention. It is accomplished with a customary, easy-to-use device. Nasal sprays have a very good user compliance.

Fucoidan should in the context of the present invention be understood as marine polysaccharides comprising L-fucose monomers, partially sulfated, that can be found in and extracted from various species of brown seaweed or brown algae (i.e. the class of Phaeophyceae). Extracts from brown seaweeds including extracts comprising fucoidan are investigated for their numerous biologic activities. As discussed in the background section, fucoidan has been screened for antiviral activity *in vitro* with mixed results. Fucoidan is not harmful to the respiratory mucosa as shown in the examples. Information on the chemical structure of fucoidan is provided in the definitions. The fucoidan may be derived from any species of brown seaweed such as *Undaria pinnatifida* or *Fucus vesiculosus.*

In one embodiment, the pharmaceutical composition is an aqueous solution. Aqueous solutions are preferred as the fucoidan is compatible with water as a carrier and stable in aqueous formulations. Aqueous solutions are especially preferred because they are compatible with the spray mechanism and the materials of most kinds of commercially available nasal spray devices. Aqueous solutions are also preferred because of the low risk of side effects.

In one embodiment, the pharmaceutical composition comprises 0.1% to 2.7%, more preferably 0.25 to 1.8%, most preferably 0.5 to 1% (w/w) fucoidan. The amounts as low as 0.1% (w/w) are sufficient to achieve the antiviral effect in the *in vitro* model and can reasonably be expected, due to the closeness of the model to the *in vivo* situation, to be effective *in vivo.* Furthermore, the amounts can be solubilised well in aqueous carriers and form stable aqueous solutions, which is beneficial for intranasal administration. With concentrations above 2.5% (w/w) of fucoidan, solubility becomes more challenging. Furthermore, pharmaceutical compositions comprising these amounts have an appearance and odour that is acceptable for nasal administration.

In one embodiment, the pharmaceutical composition comprises a sodium chloride solution. Sodium chloride may be used as a tonicity agent. Adjusting the osmolality of pharmaceutical compositions for nasal administration is beneficial to preserve the integrity of the nasal epithelia and is well-known in the art. Sodium chloride is preferred as the ions of this salt are present ubiquitously in the human body and no adverse effects are associated with intranasal administration of sodium chloride solutions. Furthermore, sodium chloride solution is also safe for ingestion and has an acceptable taste and odour. This is important for compliance, as after nasal administration, especially nasal spray administration as discussed above, the applied dose of the composition may end up in the pharyngeal region of the subject and may get into contact with the taste buds before being swallowed. It is furthermore preferred as it is compatible with fucoidan.

In one embodiment, the aqueous solution is isotonic. Isotonic solutions are preferred as they will not, when applied to the nasal epithelia, result in osmotic pressure, negative or positive, on the epithelia. They are therefore very mild carriers for the fucoidan compositions of the present invention, that preserve the integrity of the nasal epithelia.

Preferably, the isotonic aqueous solution is a sodium chloride solution, but other agents can be used to adjust osmolality.

It should be understood that in addition to the ingredients particularly mentioned above, the formulations described herein may include other agents conventional for nasal administration, such as preservatives, pH-adjusting agents, viscosity modifiers, hydrating agents, solvents, solubilisers, decongestants such as α-sympathomimetic drugs, essential oils such as peppermint oil or cooling agents such as menthol.

In one embodiment, the pharmaceutical composition is administered intranasally twice or three times daily into each nostril of a subject. The present inventors found that administration of two doses within 24 hours, eight hours apart, is more effective in reducing viral replication than application of a single dose within 24 hours, and it is therefore expected that an administration three times per day further increases efficacy. Consumers are used to applying nasal spray 3 times per day or every 8 hours, and even more frequent application, would be less consumer-friendly.

In one embodiment, the pharmaceutical composition is administered in a dose of 3.75mg to 10.15 mg fucoidan into each nostril of a subject per administration. This dose is believed to be effective *in vivo.* Transferring the doses that are effective *in vitro* to the *in vivo* situation results in a preferred daily dose of 30.3 mg/day to 60.6 mg/day fucoidan. The dose of 3.75mg to 10.15 mg fucoidan per nostril per administration takes into account the preferred dosage regimen of two times to three times daily as discussed above, and equal distribution of the total daily dose between two nostrils.

In one embodiment, the fucoidan is fucoidan extracted from *Undaria pinnatifida.* Fucoidan extracted from *Undaria pinnatifida* was described in the literature as sulfated galactofucan because it contains high amounts of galactose, such as 40 mol%, or more, of overall neutral sugars. *Undaria pinnatifida* is a preferred source for the fucoidan for the pharmaceutical compositions of the present invention as it provides fucoidans with a high degree of sulfation, and is abundantly available.

In one embodiment, the fucoidan has an average molecular weight of 150 kDa and a lower molecular weight cut-off of 10 kDa. This high average molecular weight provides for a complex structure of the polysaccharide that has a large surface for interaction with the surface of the virus. The large molecules are flexible in their configuration and may embrace the surface of the virus, allowing for multiple interactions between the virus and the fucoidan. Without being bound to a specific theory, the present inventors believe that the inhibitory effect of fucoidan on the various viruses is due to direct interaction of fucoidan with the virus. All tested virus strains bind to different host cell receptors prior to infection. The compositions of the invention are effective irrespective of the host cell receptor that a specific strain uses for cell entry. It is therefore believed that fucoidan interacts directly with the virus before binding to and infection of a host cell. The molecular weight cut-off of 10 kDa results in a higher yield of high molecular weight polymer chains. The use of high molecular weight (MW) fucoidan with the specified MW cut-off is preferred, as no uptake to systemic circulation via the mucosa is expected for high MW polymers. Even if a dose of the composition is swallowed and ingested, systemic uptake is expected to be minor, as the high molecular weight polymers are not taken up by the intestine. No systemic effects are therefore expected from intranasal administration of the pharmaceutical compositions of the invention. This is especially preferred as this reduces the risk of side effects.

In one embodiment, the fucoidan has a sulfate content of 20% to 40% (w/w). A high sulfate content comes along with a high negative charge. A high number of negatively charged residues is believed to be particularly effective. The sulfate content of a specimen of fucoidan may be determined by a gravimetric method known in the art, for example acidic hydrolysis, followed by oxidation and subsequent precipitation of barium sulfate. The determined weight for sulfate is then put in relation to the weight of the specimen of fucoidan to give the sulfate content in w/w.

### BRIEF DESCRIPTION OF THE DRAWINGS

FIG. 1: RSV-A viral genome copy numbers in copies/ml [log 10] determined in the apical culture media over time in Example 2, basal application of the compositions comprising fucoidan extracted from Fucus vesiculosus
FIG. 2: RSV-A viral genome copy numbers in copies/ml [log 10] determined in the apical culture media over time in Example 2, apical application of the compositions comprising fucoidan extracted from Fucus vesiculosus
Fig. 3: RSV-A viral genome copy numbers in copies/ml [log 10] determined in the apical culture media over time in Example 2, basal application of the compositions comprising fucoidan extracted from Undaria pinnatifida
Fig. 4: RSV-A viral genome copy numbers in copies/ml [log 10] determined in the apical culture media over time in Example 2, apical application of the compositions comprising fucoidan extracted from Undaria pinnatifida
Fig. 5: HRV-C15 viral genome copy numbers in copies/ml [log 10] determined in the apical culture media over time in Example 3, once daily apical application of the test compositions
Fig. 6: HRV-C15 viral genome copy numbers in copies/ml [log 10] determined in the apical culture media over time in Example 3, twice daily apical application of the test compositions
Fig. 7: RSV-A viral genome copy numbers in copies/ml [log 10] determined in the apical culture media over time in Example 4, once daily apical application of the test compositions
Fig. 8: RSV-A viral genome copy numbers in copies/ml [log 10] determined in the apical culture media over time in Example 4, twice daily apical application of the test compositions
Fig. 9: HRV-A16 viral genome copy numbers in copies/ml [log 10] determined in the apical culture media over time in Example 5, twice daily apical application of the test compositions
Fig. 10: HRV-B14 viral genome copy numbers in copies/ml [log 10] determined in the apical culture media over time in Example 6, twice daily apical application of the test compositions

### DETAILED DESCRIPTION OF THE INVENTION

### DEFINITIONS

The following definitions provide the meaning that should be given to specific terms in the context of the present disclosure, unless specified otherwise in specific context.

Respiratory syncytial virus (RSV) is used herein. The virus may also be known under the terms human respiratory syncytial virus (HRSV) and human orthopneumovirus, all of which are encompassed herein.

The term fucoidan specifies marine polysaccharides comprising L-fucose monomers, partially sulfated, and extracted from edible species of brown seaweed or brown algae (Phaeophyceae). The chemical structure of fucoidans is diverse and complex. Besides L-fucose, other monosaccharides such as D-fucose, mannose, galactose, glucose and xylose may be present. The configuration of the monosaccharides can vary. Glucuronic acid may also be present. The degree of sulfation, i.e. how many of the hydroxy residues of the polysaccharide are sulfated, may vary. The sulfate substitution pattern, i.e. which hydroxy groups of a monosaccharide is sulfated, may also vary. The monosaccharides may further be partly O-acetylated. The polymer may be linear or branched. The average molecular weight (MW) of fucoidan can also vary. Average MW of 4kDa to 5MDa have been reported. The average MW of fucoidan may be determined by size exclusion chromatography (SEC) such as gel permeation chromatography (GPC).

Isotonic is a solution that has an osmolarity close to that of human plasma, i.e. from 250 to 350 mOsm/L.

Normal saline is a sodium chloride solution of 0.9% m/v in purified water.

The degree of sulfation is the calculated percentage of monosaccharide units that have a sulfate substitution. The degree of sulfation may be calculated from elementary analysis to obtain the percentages of carbon and sulfur atoms in a probe and putting them in relation to an amount of carbon atoms in a galactose or fucose monosaccharide (six) and the degree of acetylation determined from that probe, obtained for example with ¹HNMR.

The sulfate content is the calculated weight percentage of sulfate groups in relation to the weight of a specimen of fucoidan. It may be determined by a gravimetric method known in the art, for example acidic hydrolysis, followed by oxidation and subsequent precipitation of barium sulfate. The determined weight for sulfate is then put in relation to the weight of the specimen of fucoidan to give the sulfate content in w/w.

### EXAMPLES

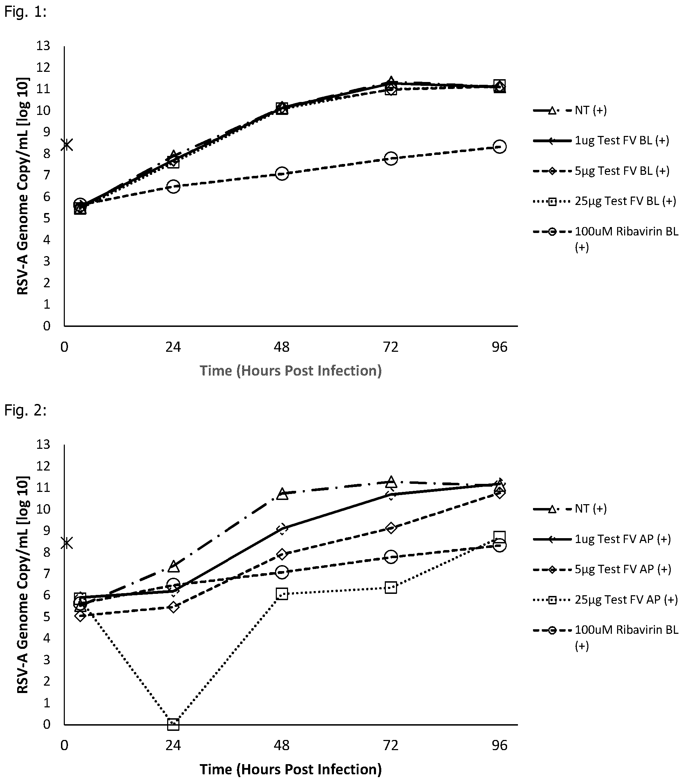

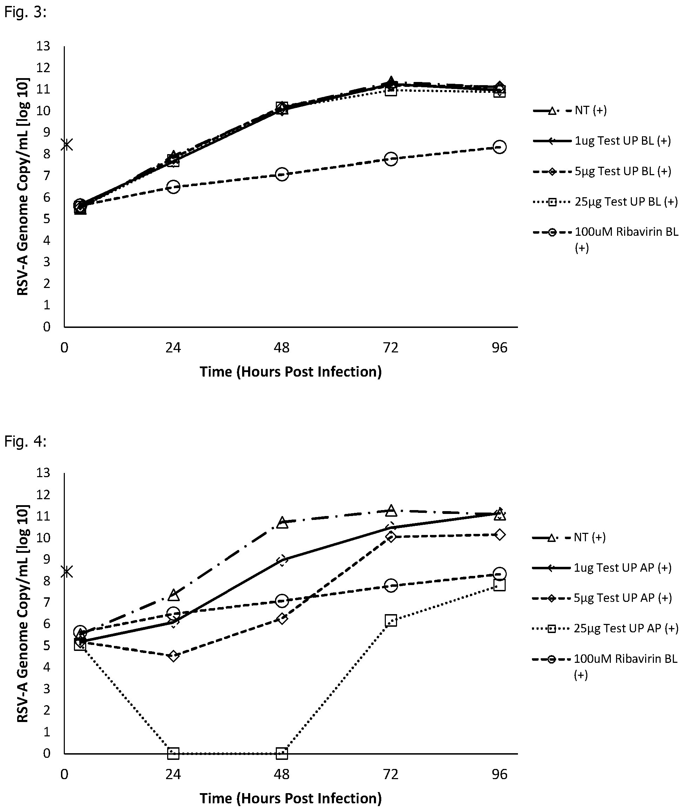

In Examples 1 and 2, the effect of compositions comprising fucoidan in different concentrations on viral genome copy numbers and indicators of epithelia integrity and inflammation in a human cell model infected with HRV-C15 (Example 1) and RSV type A (Example 2) was examined. Additionally, apical application (Figures 2 and 4) of the compositions was compared to basal application (Figures 1 and 3) and efficacy of pharmaceutical compositions comprising fucoidan sourced from two different brown algae species (*Fucus vesiculosus,* FV, and *Undaria pinnatifida,* UP) was compared.

Examples 3 and 4 compared the effect of once daily versus twice daily apical application of the pharmaceutical compositions of the invention on viral genome copy numbers and indicators of epithelia integrity and inflammation in a human cell model infected with HRV-C15 (Example 3) and RSV type A (Example 4). Additionally, efficacy of pharmaceutical compositions comprising fucoidan sourced from two different brown algae species (*Fucus vesiculosus,* FV, and *Undaria pinnatifida,* UP) was compared.

In Examples 5 and 6, the effect of twice daily apical application of the compositions of the invention on viral genome copy number in a human cell model infected with Rhinovirus-A16 (Example 5) and Rhinovirus-B14 (Example 6) was examined. Additionally, compositions of the inventions were compared to a commercially available nasal spray.

All experiments were *in vitro* experiments and conducted under the same conditions and procedures, unless indicated otherwise. Unless indicated otherwise, in the experiments, culture medium was MucilAir culture medium (product number EP04MM, can be purchased at Epithelix Sàrl, Geneva, Switzerland), which is a ready-to-use, chemically defined, serum-free culture medium. Unless specified otherwise, incubation was at 34°C, 5% CO₂ and 100% humidity.

### Cell model

The *in vitro* model used for all experiments was a standardised nasal human 3D epithelial model called MucilAir, marketed by Epithelix Sàrl, Switzerland. The model has functional characteristics similar to *in vivo* epithelia, such as mucus production, mucociliary clearance, and secretion of cytokines and chemokines and therefore is a suitable model to support the development of new antiviral pharmaceutical compositions (B. Boda et al., Antiviral drug screening by assessing epithelial functions and innate immune responses in human 3D airway epithelium model, Antiviral Research 156 (2018) 72-79, https://doi.org/10.1016/j.antiviral.2018.06.007 and A. Pizzorno et al., Characterization and treatment of SARS-CoV-2 in nasal and bronchial human airway epithelia, bioRxiv preprint, 2020, doi: https://doi.org/10.1101/2020.03.31.017889). The mature MucilAir cell model is composed of basal cells, ciliated cells and mucus cells. The proportion of these various cell types is preserved compared to what one observes *in vivo.* The cell model used for the experiment was obtained as follows: Airway cells were obtained from patients undergoing nasal biopsy. Human airway epithelial cells were isolated, amplified and expanded by two passages to preserve the physiological characteristics of the cells. The cells were seeded onto a semi-porous membrane (Costar Transwell, pore size 0.4 µm) and cultured at the air-liquid interface for differentiation. Airway epithelia was reconstituted from a mixture of human airway cells of 14 individual healthy donors to lessen differences between donors (technique called MucilAir-Pool). The reconstituted airway epithelia was cultured at the air-liquid interface (ALI) in culture medium in 24-well plates with 6.5-mm Transwell inserts (cat #3470, Corning Incorporated, Tweksbury, USA). All used inserts were fully differentiated at the start of the experiments. Three days before the start of the experiments, the integrity and quality of all used cell culture inserts used was ensured by a three-step quality assurance test. Firstly, visual inspection under a conventional inverted microscope was performed. The pass criteria were visible cilia beating and uniform and homogenous appearance. Secondly, it was ensured that all inserts showed physiological mucus production by measuring the refractive index of the apical surface of the inserts. Thirdly, trans epithelial electronic resistance (TEER) was measured as described later in the description. Pass criteria was a TEER value of more than 200 Ω.cm². All used inserts complied with the quality assurance test. Each insert was then, still on the same day, washed apically with culture medium to remove accumulated mucus and cell debris to minimize the risk of interference with the tests.

### Compositions

The compositions used in the experiments are specified in Tables 1 - 3 below:

**Table 1: Compositions used in Example 1 and Example 2**

| Composition | Main component | Concentration of main component | Details of main component | Dose and application |
|---|---|---|---|---|
| 25 µg Test FV AP | Fucoidan | 2500 µg/ml in normal saline | Fucoidan powder from *Fucus vesiculosus,* product Maritech Synergy (Marinova) | 10 µl, apically |
| 5 µg Test FV AP | Fucoidan | 500 µg/ml in normal saline | As 25 µg Test FV AP | 10 µl, apically |
| 1 µg Test FV AP | Fucoidan | 100 µg/ml in normal saline | As 25 µg Test FV AP | 10 µl, apically |
| 25 µg Test FV BL | Fucoidan | 50 µg/ml in culture medium | As 25 µg Test FV AP | 500 µl, basal |
| 5 µg Test FV BL | Fucoidan | 10 µg/ml in culture medium | As 25 µg Test FV AP | 500 µl, basal |
| 1 µg Test FV BL | Fucoidan | 2 µg/ml in culture medium | As 25 µg Test FV AP | 500 µl, basal |
| 25 µg Test UP AP | Fucoidan | 2500 µg/ml in normal saline | Fucoidan powder from *Undaria pinnatifida,* product MariUP (Marinova) | 10 µl, apically |
| 5 µg Test UP AP | Fucoidan | 500 µg/ml in normal saline | As 25 µg Test UP AP | 10 µl, apically |
| 1 µg Test UP AP | Fucoidan | 100 µg/ml in normal saline | As 25 µg Test UP AP | 10 µl, apically |
| 25 µg Test UP BL | Fucoidan | 50 µg/ml in culture medium | As 25 µg Test UP Ap | 500 µl, basal |
| 5 µg Test UP BL | Fucoidan | 10 µg/ml in culture medium | As 25 µg Test UP Ap | 500 µl, basal |
| 1 µg Test UP BL | Fucoidan | 2 µg/ml in culture medium | As 25 µg Test UP Ap | 500 µl, basal |
| Positive control for Rhinovirus (Example 1) | rupintrivir | 5 µM drug in DMSO solution with water, final DMSO concentration 0.25% | Purchased from Santa Cruz Biotechnology (Dallas, US) | added to basal culture medium |
| Positive control for respiratory syncytial virus (Example 2) | ribavirin | 100 µM drug in DMSO solution with water, final DMSO concentration 0.24% | Purchased from Sigma | added to basal culture medium |
| Negative basolateral control | Culture medium | 100% | MucilAir culture medium (Epithelix Sàrl, Geneva, Switzerland). | 500 µl, basolateral |
| Negative Control (vehicle) | Normal saline | - | - | 10 µl, apically |
| Positive control for cytotoxicity: Triton X-100 | Triton X-100 | 10% (v/v) in normal saline | - | 50 µl, apically |
| Positive control for inflammatory response: Cytomix | Cytomix | 500 ng/ml TNFα, 0.2 mg/ml LPS, 1 % FCS in MucilAir culture medium | - | 100 µl basolateral |

**Table 2: Compositions used in Example 3 and Example 4**

| Composition | Main component | Concentration of main component | Details of main component | Dose and application |
|---|---|---|---|---|
| 25 µg Test FV | Fucoidan | 2500 µg/ml in normal saline | Same as 25 µg Test FV AP in Examples 1 and 2 | 10 µl, apically |
| 25 µg Test UP | Fucoidan | 2500 µg/ml in normal saline | Same as 25 µg Test FV AP in Examples 1 and 2 | 10 µl, apically |
| Positive antiviral control for rhinovirus, positive antiviral control for respiratory syncytial virus, negative control, positive control for cytotoxicity and positive control for inflammatory response were the same as in Examples 1 and 2. | | | | |

**Table 3: Compositions used in Example 5 and Example 6**

| Composition | Main component | Concentration of main component | Details of main component | Dose and application |
|---|---|---|---|---|
| 200 µg Test UP | Fucoidan | 20000 µg/ml in normal saline | Same as 25 µg Test FV AP Examples 1 and 2 | 10 µl, apically |
| 100 µg Test UP | Fucoidan | 10000 µg/ml in normal saline | As 200 µg Test UP | 10 µl, apically |
| 50 µg Test UP | Fucoidan | 5000 µg/ml in normal saline | As 200 µg Test UP | 10 µl, apically |
| 25 µg Test UP | Fucoidan | 2500 µg/ml in normal saline | As 200 µg Test UP | 10 µl, apically |
| 12.5 µg Test UP 1250 | Fucoidan | 1250 µg/ml in normal saline | As 200 µg Test UP | 10 µl, apically |
| Boots Dual Defense | Iota-carrageenan and kappa-carrageenan | 1600 µg/ml solution (1200 µg/ml Iota-carrageenan + 400 µg/ml K-carrageenan | Commercially available nasal spray | 10 µl, apically |
| Positive antiviral control for rhinovirus was rupintrivir as in Examples 1 and 3. Negative control, positive control for cytotoxicity and positive control for inflammatory response were the same as in Examples 1 to 4. | | | | |

All fucoidan compositions were produced by mixing the fucoidan powder with normal saline in a concentration of 10 mg/ml and solubilisation by vortexing, and optional further dilution with normal saline (0.9% w/v NaCl in purified water) by volume for the test compositions with lower concentrations. Only 200 µg Test UP was directly produced by solubilising by vortexing 20 mg fucoidan powder in 1 ml normal saline to give the concentration of 20000 µg/ml. For apical application of the fucoidan compositions, a volume of 10 µl was pipetted onto the apical side of the cell model. This was performed twice daily or once daily. For basal application of the fucoidan compositions in Examples 1 and 2, a volume of 500 µl of solutions with lower concentrations was added to the basal cell culture medium of the cell model. Thereby, equal doses of fucoidan were applied to the apical and basolateral compartment and the results could be compared. Because the basal cell culture medium was renewed every 24 hours, this was repeated after addition of the fresh cell culture medium. The Boots Dual Defense product was applied by pipetting of a volume of 10 µl onto the apical side of the cell model.

Positive control for inhibition of virus replication were solutions of the antiviral drugs rupintrivir for experiments with rhinovirus strains (Examples 1, 3, 5 and 6) and of ribavirin for experiments with RSV A (Examples 2 and 4). Rupintrivir was purchased from Santa Cruz Biotechnology (Dallas, US) and ribavirin was purchased from Sigma. Both drugs were diluted in DMSO and water, to a final concentration of DMSO of 0.25% for both and a final drug concentration of 5 µM of rupintrivir or 100 µM of ribavirin. The positive control antiviral drug solutions were added to the basal cell culture medium, concomitantly with viral inoculation, which was performed from the apical side. Basal media of the cell cultures was changed every 24 hours and the antiviral drug solutions were subsequently added to the fresh basal culture medium. In Examples 1 and 2, a negative control of 1% DMSO in normal saline was carried out, apically and from the basal side. It showed that this solvent mixture, both from the apical and basal side, has no impact on the parameters measured in the experiment. [data not shown]

Positive control for cytotoxicity in all experiments was Triton X-100 (Polyethylene glycol tert-octyl phenyl ether), a non-ionic surfactant often used in biochemical applications to permeabilise or lyse cell membranes. 50 µl at a concentration of 10% (v/v) in normal saline were pipetted onto the apical side of the inserts. Triton X-100 causes a massive LDH release in reconstituted human airway epithelia and was used as 100% cytotoxicity landmark. Positive control for inflammatory response was in all experiments Cytomix.

As a negative control, 10 µl normal saline were applied to the apical side of the cell cultures in all experiments.

### Virus inoculation

Four respiratory viruses were used in separate and subsequentially performed experiments:
HRV-C15 was used for Examples 1 and 3 in a concentration of 10⁶ genome copies number/ml and each insert was inoculated with 10⁵ genome copies in 100µl of culture medium.
RSV-A was used for Examples 2 and 4 in a concentration of 10⁶ genome copies number/ml and each insert was inoculated with 10⁵ genome copies in 100µl of culture medium.
HRV-A16 was used for Example 5 in a concentration of 10⁶ genome copies number/ml and each insert was inoculated with 10⁵ genome copies in 100µl of culture medium.
HRV-B14 was used for Example 6 in a concentration of 10⁶ genome copies number/ml and each insert was inoculated with 10⁵ genome copies in 100µl of culture medium.
Rhinovirus C15 (isolation year 2009) and respiratory syncytial virus A (isolation year 2017, Switzerland) were clinical specimens characterized and described by Essaidi-Laziosi et al., 2018, whereas rhinovirus HRV-A16 and HRV-B14 were purchased from American Type Culture Collection (VR-283 and VR-284). Viral stocks for the experiments were produced in MucilAir cell cultures beforehand and collected from apical washes of the cell culture with culture medium. The yield of several days was pooled and quantified by qPCR, aliquoted and stored at -80 °C. Inoculation of the cell cultures was performed on day 1 of the experiments. Prior to infection, the apical side of the inserts used for the experiments was washed once for 10 minutes with culture medium. Inoculations were performed with 100 µl of culture medium containing 10⁵ viral particles applied to the apical side of the cultures (the virus concentration was 10⁶/ml) and incubated for 3 hours at 34°C, 5 % CO₂. The viral solution used for the inoculation was re-quantified by qPCR and showed that the actual viral concentration in some inoculums was higher or lower than expected:The concentration of RSV A in Example 2 was 10⁸/ml, the concentration of HRV-C15 in Example 3 was 1.1x10⁷/ml, the concentration of RSV A in Example 4 was 8.2x10⁸/ml, the concentration of HRV-A16 in Example 5 was 7.1x10⁵/ml and the concentration of HRV-B14 in Example 6 was 2.6x10⁵/ml. Non-infected control inserts ("Mock") were exposed to 100 µl of culture medium without virus on the apical side for 3 hours at 34 °C, 5 % CO₂. Unbound viruses were washed away with culture medium after the 3 hours of incubation period by three rapid washing steps. Residual viruses after the 3 washes were collected by a 20 min apical wash and quantified by qPCR to establish a baseline for viral growth at later time points. New viral particles from replication in the infected cell cultures were collected by 20 minutes apical washes at 24, 48, 72 and 96 hours post-inoculation and quantified by qPCR.

### Test parameters

Viral genome copy numbers are a direct measure for the number of viral particles present in a sample. In the experiments, viral genome copy numbers of the cell cultures treated with the different compositions or untreated were compared, to establish the effect of the different interventions on the viral replication. To obtain the viral genome copy numbers, 20 µl of the 200 µl of apical washing liquid were used for viral RNA extraction with the QIAamp Viral RNA kit (Qiagen), obtaining 60 µl of eluted RNA. Viral RNA was then quantified by quantitative RT-PCR (QuantiTect Probe RT-PCR, Qiagen) using 5 µl of viral RNA with Mastermix and three Picornavirus specific (experiments with rhinoviruses, i.e. Examples 1, 3, 5 and 6) or two RSV-A specific (experiments with RSV, i.e. Examples 2 and 4) primers and probe with FAM-TAMRA reporter-quencher dyes. Four dilutions of known concentration of HRV-A16 (experiments with rhinoviruses, i.e. Examples 1, 3, 5 and 6) or RSV-A RNAs (experiments with RSV, i.e. Examples 2 and 4) as well as control for RT-PCR were included and the plates were run on a Chromo4 PCR Detection System from Bio-Rad. Cycle threshold data were reported to the standard curve, corrected with the dilution factor and presented as genome copy number per ml on the graphs.

Trans-epithelial electronic resistance (TEER) is a dynamic parameter that reflects the tissue integrity of epithelia and is typically between 200 to 600 Ω.cm² in the used cell model. An increase of the TEER value reflects a blockage of the ion channel activities of the epithelial cells. A notable decrease of the TEER values, but with the value still above 100 Ω.cm² indicates an activation of the ion channels. Disruption of cellular junction or holes in the epithelia result in TEER values below 100 Ω.cm². When an epithelium is damaged, a decrease of TEER would be associated with an increase of LDH release or a decrease of the cell viability. In the examples, TEER was measured after addition of 250 µl of normal saline in Examples 1 and 2, or 200 µl of culture medium in Examples 3, 4, 5 and 6, to the apical compartment of the inserts (i.e. during the washing step). TEER was measured with an EVOMX volt-ohm-meter (World Precision Instruments UK, Stevenage) for each condition. Resistance values (Ω) were converted to TEER (Ω.cm²) using the following formula: TEER (Ω.cm²) = (resistance value (Ω) - 100 (Ω)) x 0.33 (cm²), where 100 Ω is the resistance of the membrane and 0.33 cm² is the total surface of the epithelium in one insert. The TEER measurement was conducted as follows: 200µl of culture medium or 250 µl of normal saline (only in Examples 1 and 2) was added on apical surface of each insert. The EVOMX was turned on. The electrode was washed with ethanol 70%, and the EVOMX screen showed the value -1. Then, the electrode was washed with culture medium, and the EVOMX screen showed 0.00. Then, the resistance (Ω) was measured with the EVOMX in the Ohms measurement function.

Lactate dehydrogenase (LDH) is a stable cytoplasmic enzyme that is rapidly released into the basal culture medium upon rupture of the plasma membrane. In the experiments, it served as a parameter indicating cytotoxic effect of the interventions. It was measured using the Cytotoxicity LDH Assay Kit-WST (Dojindo, CK12-20) which was read out using a plate reader to measure the absorbance of the samples at 490 nm. Samples were 50 µl of the basal cell culture medium collected from the inserts at each time point. To determine the percentage of cytotoxicity, the following equation was used (A = absorbance values): Cytotoxicity (%) = (Aₓ (determined absorbance of the sample)-A_{L} (absorbance of the low control)/A_{H} (absorbance of the high control)-A_{L} (low control))^{∗}100. The high control was the positive control for cytotoxicity (10 % Triton X-100 apical treatment). Triton X-100 causes a massive LDH release and corresponds to 100 % cytotoxicity. The low control was basal culture medium of fresh MucilAir cell inserts. The negative controls (non-treated and vehicle) show a low daily basal LDH release of <5 %, which is indicates a physiological cell turnover in the cell model.

Cilia beating frequency (CBF) is a parameter that indicates if the nasal epithelia is healthy and carrying out its physiologic function of mucus transport. CBF was measured by a dedicated setup for this purpose. The system consists of three parts: a Sony XCD V60 camera connected to an Olympus BX51 microscope, a PCI card and a specific package of software. The cilia beating frequency is expressed in Hz. For measurement, a cell culture insert was placed under the microscope and 256 images were captured at high frequency rate (125 frames per second) at 34°C. CBF was then calculated using relevant software. It should be pointed out that CBF values may be subject to fluctuations due to parameters such as temperature, mucus viscosity or liquid applied on the apical surface the cell model and observed values should be compared to a control condition in each experiment.

Mucociliary clearance (MCC) results from synchronised cilia-beating and also is a parameter that indicates if the nasal epithelia is healthy and carries out its physiologic function of mucus transport. MCC was monitored using a Sony XCD-U100CR camera connected to an Olympus BX51 microscope with a 5x objective. Polystyrene microbeads of 30 µm diameter (Sigma, 84135) were added on the apical surface of the cell cultures. Microbeads movements were video tracked at 2 frames per second for 30 images at 34°C. Three movies were taken per insert. Average beads movement velocity (µm/sec) was calculated with the ImageProPlus 6.0 software.

Three biomarkers (Interleukin 8 (IL-8), Interleukin 6 (IL-6), and RANTES) were measured in the experiments using commercially available ELISA assays. Release of chemokines and cytokines was measured according to the manufacturer's instructions with the ELISA assays. Lyophilised standards were reconstituted, aliquoted and stored at -80°C. Each ELISA plate contained a standard curve. Samples were diluted with the appropriate assay diluent at the appropriate dilution rate, washing steps were performed with an automatic microplate washer and absorbance was measured at 450 nm.

Table 4 gives details about the cytokine and chemokine biomarkers and the used material. These biomarkers were selected as they are known to be modulated upon viral infections of the reconstituted human airway epithelia (Essaidi-Laziosi M. et al., http://dx.doi.org/10.1016/j.jaci.2017.07.018)

**Table 4: Biomarkers and material**

| **Name** | **Description** | **Material** |
|---|---|---|
| Interleukin 8 (or CXCL8) | Chemokine (C-X-C motif) Ligand 8; neutrophil chemotactic factor | BD Biosciences 555244 (detection: 3-200 pg/ml) |
| Interleukin 6 | Pro-inflammatory cytokine | BD Biosciences 555220 (detection: 3-300 pg/ml) |
| RANTES | Chemokine (C-C motif) ligand 5, CCL5 or regulated on activation, normal T cell expressed and secreted | R&D Systems, DY278 (detection: 15.6-1000 pg/ml) |

### Statistical Analysis

Data were expressed as mean±standard deviation and assumed to have normal distribution. Differences between three or more groups were tested by one-way or two-way ANOVA with Dunnett's multiple comparison post-tests using Prism 6 GraphPad software (La Jolla, USA). Differences between two groups were tested by Student's t test. The values P<0.05 were considered statistically significant.

### Example 1

### Experimental setup

In Example 1, each intervention was performed in three different inserts (i.e. three replicates). Consequently, three inserts each were treated with the different fucoidan test compositions apically or from the basal side. The following negative controls were performed: Three inserts were not inoculated with virus and not treated ("Mock"). Six inserts were not inoculated with virus but were treated with the two test compositions in the highest concentration (25 µg Test FV AP and 25 µg Test UP AP) apically. Six inserts were not inoculated with virus but were treated with the two test compositions in the highest concentration (25 µg Test FV BL and 25 µg Test UP BL) from the basal side. Three inserts were inoculated but not treated. Three inserts were inoculated and treated with 10 µl cell saline apically (negative control). Three inserts were inoculated and treated with 500 µl cell culture medium added to the basal compartment (NT (+)). Three inserts were treated with 10 µl 0.25%DMSO (apical) in normal saline and three inserts were treated with 500 µl 0.25%DMSO in normal saline as apical and basal control for the vehicle of the antiviral drugs. The following positive controls were performed: Three inserts were treated with 5µM positive antiviral control (rupintrivir) added to the basal cell culture medium. Three inserts were treated with 50 µl apical positive control for cytotoxicity (Triton X-100). Three inserts were treated with basal positive control for inflammatory response (Cytomix).

### Protocol

At the start of the experiment (T0), the inserts were washed with 200 µl culture medium for 10 minutes at 34°C and the inserts were transferred into a new culture plate containing 500 µl of culture medium per well. To the wells of the inserts to be treated from the basal side, additionally 500 µl of the basal test compositions were added (25 µg Test FV BL, 5 µg Test FV BL, 1 µg Test FV BL, 25 µg Test UP BL, 5 µg Test UP BL, 1 µg Test UP BL), according to the experimental setup. 10 µl of apical test compositions (25 µg Test FV AP, 5 µg Test FV AP, 1 µg Test FV AP, 25 µg Test UP AP, 5 µg Test UP AP, 1 µg Test UP AP), were pipetted onto the apical side of all inserts to be treated according to the experimental setup. All inserts were incubated for 1 hour.

At T1h, inserts to be inoculated according to the experimental setup were inoculated as described above with HRV-C15. Inserts not to be inoculated according to the experimental setup ("Mock") were instead exposed to 100 µl of culture medium on the apical side. Some inserts were additionally treated with positive antiviral control (rupintrivir) from the basal side as foreseen in the experimental setup. All inserts were incubated for 3 hours.

At T4h, all inserts were washed twice with phosphate buffered saline (with Ca²⁺/Mg²⁺) at 34°C to remove the viral inoculum and unbound viruses. Afterwards, 200 µl of culture medium was added apically to all inserts and inserts were let sit for 20 minutes at 34°C. This apical washing liquid was then removed and was stored at -80°C until the virus copy number was determined. 10 µl of the apical test compositions (25 µg Test FV AP, 5 µg Test FV AP, 1 µg Test FV AP, 25 µg Test UP AP, 5 µg Test UP AP, 1 µg Test UP AP) were pipetted onto the apical side of all inserts to be treated apically according to the experimental setup.

At T24h, 200 µl of culture medium was added apically to all inserts and inserts were let sit for 20 minutes at 34°C. During this time, TEER of all inserts was measured. This apical liquid was then removed and was stored at -80°C until the virus copy number was determined. 10 µl of the apical test compositions (25 µg Test FV AP, 5 µg Test FV AP, 1 µg Test FV AP, 25 µg Test UP AP, 5 µg Test UP AP, 1 µg Test UP AP) were pipetted onto the apical side of all inserts to be treated apically according to the experimental setup. The basal culture medium was removed and stored. All inserts were transferred to new culture plates containing 500 µl culture media per well, and wells of the inserts to be treated from the basal side additionally containing 500 µl of the basal test compositions (25 µg Test FV BL, 5 µg Test FV BL, 1 µg Test FV BL, 25 µg Test UP BL, 5 µg Test UP BL, 1 µg Test UP BL), according to the experimental setup. Some inserts were additionally treated with positive antiviral control (rupintrivir) from the basal side as foreseen in the experimental setup.

At T48h, 200 µl of culture medium was added apically to all inserts and inserts were let sit for 20 minutes at 34°C. During this time, TEER of all inserts was measured. This apical liquid was then removed and was stored at -80°C until the virus copy number was determined. The basal culture medium was removed and stored for the measurement of the biomarkers that was conducted later. All inserts were transferred to new culture plates containing 500 µl culture media per well, and wells of the inserts to be treated from the basal side additionally containing 500 µl of the basal test compositions (25 µg Test FV BL, 5 µg Test FV BL, 1 µg Test FV BL, 25 µg Test UP BL, 5 µg Test UP BL, 1 µg Test UP BL), according to the experimental setup. Some inserts were additionally treated with positive antiviral control (rupintrivir) from the basal side as foreseen in the experimental setup. 10 µl of the apical test compositions (25 µg Test FV AP, 5 µg Test FV AP, 1 µg Test FV AP, 25 µg Test UP AP, 5 µg Test UP AP, 1 µg Test UP AP) were pipetted onto the apical side of all inserts to be treated apically according to the experimental setup. All inserts were incubated for 24 hours.

At T72h, 200 µl of culture medium was added apically to all inserts and inserts were let sit for 20 minutes at 34°C. During this time, TEER of all inserts was measured. This apical liquid was then removed and was stored at -80°C until the virus copy number was determined. 10 µl of the apical test compositions (25 µg Test FV AP, 5 µg Test FV AP, 1 µg Test FV AP, 25 µg Test UP AP, 5 µg Test UP AP, 1 µg Test UP AP) were pipetted onto the apical side of all inserts to be treated apically according to the experimental setup. The basal culture medium was removed and stored. All inserts were transferred to new culture plates containing 500 µl culture media per well, and wells of the inserts to be treated from the basal side additionally containing 500 µl of the basal test compositions (25 µg Test FV BL, 5 µg Test FV BL, 1 µg Test FV BL, 25 µg Test UP BL, 5 µg Test UP BL, 1 µg Test UP BL), according to the experimental setup. Some inserts were additionally treated with positive antiviral control (rupintrivir) from the basal side as foreseen in the experimental setup. All inserts were incubated for 24 hours.

At T96h, the ciliary beating frequency (CBF) and the mucociliary clearance (MCC) of all inserts were measured. 200 µl of culture medium was added apically to all inserts and inserts were let sit for 20 minutes at 34°C. During this time, TEER of all inserts was measured. This apical liquid was then removed and was stored at -80°C until the virus copy number was determined. The basal culture medium was removed and stored for the measurement of the biomarkers that was conducted later.

### Results

An overview of the qualitative results of Example 1 for all parameters is shown in Table 5.

Firstly, basal antiviral positive control (rupintrivir) was effective and decreased the viral load on average by two logs. This shows that the drug at the used concentration is a suitable positive antiviral control for HRV-C15 in the cell model. The experiment further showed that basal exposure with the fucoidan compositions did not reduce the viral replication, whereas application of the fucoidan compositions to the apical side of the cell model could inhibit or delay the viral replication. 25 µg Test UP AP was the most effective composition. The results confirm that intranasal administration of the compositions of the invention is effective in reducing viral genome copy number of HRV-C15 in a dose dependent manner and that application from the apical side, i.e. for example a nasal spray, is a suitable form of administration.

Trans-epithelial electronic resistance (TEER) was measured at 24, 48, 72 and 96 hours. Treatment with Triton X-100 resulted in a harshly reduced TEER below 100 Ω.cm², and therefore was a suitable positive control agent for a negative impact on tissue integrity. Inserts that were not inoculated with virus but treated apically with fucoidan test compositions in the different concentrations (25 µg Test FV AP, 5 µg Test FV AP, 1 µg Test FV AP, 25 µg Test UP AP, 5 µg Test UP AP, 1 µg Test UP AP) showed TEER values in the normal range of the cell model. These concentrations of the compositions therefore are believed to be highly compatible with human nasal epithelia and to have no negative impact on tissue integrity. Inserts inoculated with HRV-C15 showed significantly reduced TEER values at day three post inoculation. TEER values subsequently recovered to normal values. While the positive control, rupintrivir, prevented this transient TEER decrease, the apical treatment with fucoidan compositions (25 µg Test FV AP, 5 µg Test FV AP, 1 µg Test FV AP, 25 µg Test UP AP, 5 µg Test UP AP, 1 µg Test UP AP) did not modify it. Inserts treated with basal application of *Fucus vesiculosus* compositions (25 µg Test FV BL, 5 µg Test FV BL or 1 µg) showed more variable TEER values and no clear dose dependent change was observed, whereas basal application of *Undaria pinnatifida* compositions seemed to diminish the HRV-C15 induced TEER fluctuation.

As per the measurement scheme used for cytotoxicity explained above, LDH levels of Triton X-100 treated inserts was set to be 100% cytotoxicity, and the LDH levels measured in all other inserts were put in relation to this value. All cell culture inserts treated (except for the positive control) at all measured timepoints showed less than 5% cytotoxicity.RV-C15 thus does not have cytotoxic effects on the cell model in the observed timeframe. This result shows that the compositions do not have cytotoxic effects and may be used *in vivo* as nasal spray compositions for humans without negative effects on the nasal epithelia.

IL-8 levels were measured on day 2 and day 4 and showed that, as expected, cytomix induced a high and significant increase in IL-8. Inserts that were not infected but treated apically with fucoidan test compositions showed IL-8-levels comparable to those of the not infected, untreated inserts (Mock). This shows that the compositions do not provoke inflammatory response in nasal epithelia and are safe for use as a nasal spray in humans. RV-C15 in inoculated inserts, treated with vehicle, induced a significant increase of IL-8 release at day 4 post infection. This effect could be prevented by the positive control treatment with rupintrivir. Treatment with fucoidan compositions, both in apical and basal application, did statistically significant reduce the IL-8 release on day 4.

IL-6 levels were measured on day 2 and day 4. As expected, cytomix induced an IL-6 release. In the inserts that were not infected, treatment with the test compositions showed even lower IL-6 release than the untreated and uninfected inserts (mock) on both days. HRV-C15 infection induced a significant high release of IL-6 on day 4. This virus-triggered IL-6 level on day 4 was significantly reduced by treatment with 25 µg Test FV AP, and by all UP compositions (25 µg, 5 µg and 1 µg UP, AP and BL). 25 µg Test UP AP was the most effective composition to lower the IL-6 release.

RANTES levels were measured on day 2 and day 4. As expected, cytomix induced a mild increase of RANTES at day 4. In the inserts that were not infected, treatment with the test compositions showed approximately the same RANTES levels as the untreated and uninfected inserts (mock). Only 25 µg Test FV AP induced a very mild increase of RANTES at day 4. HRV-C15 infection induced a significant and high release of RANTES on day 4. This virus-triggered RANTES level on day 4 was diminished slightly, but significantly by treatment with 25 µg Test FV AP and 25 µg Test FV BL. Treatment with UP compositions did not change RANTES levels significantly.

**Table 5: Overview of results of Example 1;**

| Example 1 | | | | | | |
|---|---|---|---|---|---|---|
| | Virus induced deregulation | FV compositions | | UP compositions | | Positive antiviral control |
| | | BL | AP | BL | AP | |
| Viral genome copy number | | - | ↓(2logs) | - | ↓ (2 and 4 logs) | ↓ (3logs) |
| TEER | ↓ | - | - | attenuation | - | prevention |
| LDH | - | - | - | - | - | - |
| CBF | ↓ | ↑ | - | ↑ | - | prevention |
| MCC | ↓ | - | - | - | - | - |
| IL-8 | ↑ | ↓ | ↓ | ↓ | ↓ | ↓ |
| IL-6 | ↑ | ↓ | - | ↓ | ↓ | ↓ |
| RANTES | ↑ | ↓ | ↓ | - | - | ↓ |

| | | | | | | |
|---|---|---|---|---|---|---|
| ↑ : increase, ↓ : decrease, with the magnitude indicated in brackets, - : no effect | | | | | | |

### Example 2

The compositions used in Example 2 are indicated in Table 1. The experimental setup and the protocol of Example 2 were the same as for Example 1, except that respiratory syncytial virus A was used as viral inoculum, and consequently ribavirin was used as positive antiviral control.

### Results

The effect of the different interventions on the viral genome copy number of RSV A is visualised in Figure1 1-4 and the results are given in Table 6. An overview of the results for all parameters is shown in Table 7.

**Table 6: Results of the measurement of the viral genome copy number in Example 2**

| | Mean Log 10 ((Copies/ml) + 1) | | | | |
|---|---|---|---|---|---|
| Hours post infection | NT | 1ug Test FV BL | 5µg Test FV BL | 25µg Test FV BL | 100uM Ribavirin BL |
| 3,5 | 5,50 | 5,57 | 5,50 | 5,45 | 5,63 |
| 24 | 7,90 | 7,69 | 7,70 | 7,59 | 6,48 |
| 48 | 10,13 | 10,12 | 10,16 | 10,08 | 7,07 |
| 72 | 11,34 | 11,27 | 10,98 | 11,00 | 7,78 |
| 96 | 11,11 | 11,10 | 11,13 | 11,16 | 8,32 |

| | Standard Deviation Log 10 ((Copies/ml) + 1) | | | | |
|---|---|---|---|---|---|
| 3,5 | 4,57 | 5,01 | 5,02 | 4,30 | 5,03 |
| 24 | 7,36 | 7,15 | 7,24 | 7,02 | 5,79 |
| 48 | 9,41 | 8,94 | 8,91 | 8,93 | 6,73 |
| 72 | 10,60 | 10,77 | 10,20 | 10,39 | 7,41 |
| 96 | 10,11 | 9,94 | 9,72 | 9,97 | 8,31 |

| | Mean Log 10 ((Copies/ml) + 1) | | | | |
|---|---|---|---|---|---|
| | NT (+) | 1ug Test FV AP (+) | 5µg Test FV AP (+) | 25µg Test FV AP (+) | 100uM Ribavirin BL (+) |
| 3,5 | 5,52 | 5,90 | 5,06 | 5,83 | 5,63 |
| 24 | 7,37 | 6,21 | 5,46 | 0,00 | 6,48 |
| 48 | 10,73 | 9,09 | 7,91 | 6,06 | 7,07 |
| 72 | 11,28 | 10,69 | 9,13 | 6,36 | 7,78 |
| 96 | 11,10 | 11,18 | 10,76 | 8,69 | 8,32 |

| | Standard Deviation Log 10 ((Copies/ml) + 1) | | | | |
|---|---|---|---|---|---|
| 3,5 | 4,51 | 6,04 | 4,77 | 6,00 | 5,03 |
| 24 | 6,75 | 5,66 | 4,81 | 0,00 | 5,79 |
| 48 | 10,06 | 8,54 | 7,18 | 6,11 | 6,73 |
| 72 | 10,73 | 10,31 | 9,30 | 6,24 | 7,41 |
| 96 | 10,07 | 10,37 | 9,80 | 8,35 | 8,31 |

| | Mean Log 10 ((Copies/ml) + 1) | | | | |
|---|---|---|---|---|---|
| | NT | 1uq Test UP BL | 5µg Test UP BL | 25µg Test UP BL | 100uM Ribavirin BL |
| 3,5 | 5,50 | 5,68 | 5,54 | 5,54 | 5,63 |
| 24 | 7,90 | 7,67 | 7,82 | 7,69 | 6,48 |
| 48 | 10,13 | 10,05 | 10,18 | 10,13 | 7,07 |
| 72 | 11,34 | 11,25 | 11,19 | 10,96 | 7,78 |
| 96 | 11,11 | 10,97 | 11,12 | 10,89 | 8,32 |

| | Standard Deviation Log 10 ((Copies/ml) + 1) | | | | |
|---|---|---|---|---|---|
| 3,5 | 4,57 | 4,44 | 4,75 | 4,93 | 5,03 |
| 24 | 7,36 | 7,12 | 6,46 | 7,26 | 5,79 |
| 48 | 9,41 | 9,59 | 9,45 | 9,05 | 6,73 |
| 72 | 10,60 | 10,37 | 10,42 | 9,59 | 7,41 |
| 96 | 10,11 | 10,21 | 9,95 | 10,69 | 8,31 |

| | Mean Log 10 ((Copies/ml) + 1) | | | | |
|---|---|---|---|---|---|
| | NT (+) | 1ug Test UP AP (+) | 5µg Test UP AP (+) | 25µg Test UP AP (+) | 100uM Ribavirin BL (+) |
| 3,5 | 3,52 | 0,00 | 0,00 | 0,00 | 3,52 |
| 24 | 10,22 | 7,67 | 5,99 | 5,14 | 10,22 |
| 48 | 11,46 | 9,89 | 8,48 | 7,63 | 11,46 |
| 72 | 10,33 | 8,53 | 6,96 | 6,89 | 10,33 |
| 96 | 9,09 | 6,86 | 5,48 | 6,24 | 9,09 |

| | Standard Deviation Log 10 ((Copies/ml) + 1) | | | | |
|---|---|---|---|---|---|
| 3,5 | 4,51 | 4,50 | 4,67 | 4,35 | 5,03 |
| 24 | 6,75 | 5,69 | 4,40 | 0,00 | 5,79 |
| 48 | 10,06 | 8,60 | 5,96 | 0,00 | 6,73 |
| 72 | 10,73 | 9,99 | 9,72 | 5,84 | 7,41 |
| 96 | 10,07 | 9,99 | 9,94 | 7,62 | 8,31 |

When the viral genome copy number was below the detection limit of the method, it was noted as 0.00 in the table and expressed as log 10 ((copies/ml) + 1) in the graphs. The number 1 was added to include values of 0.00 copies/ml in the graphs. In Fig. 1-4, NT stands for negative control inserts for basal application, i.e. inserts infected and treated with 500 µl culture medium, added to basal culture medium. Vehicle AP stands for negative control inserts for apical application, i.e. inserts infected and treated with 10 µl culture medium apically.

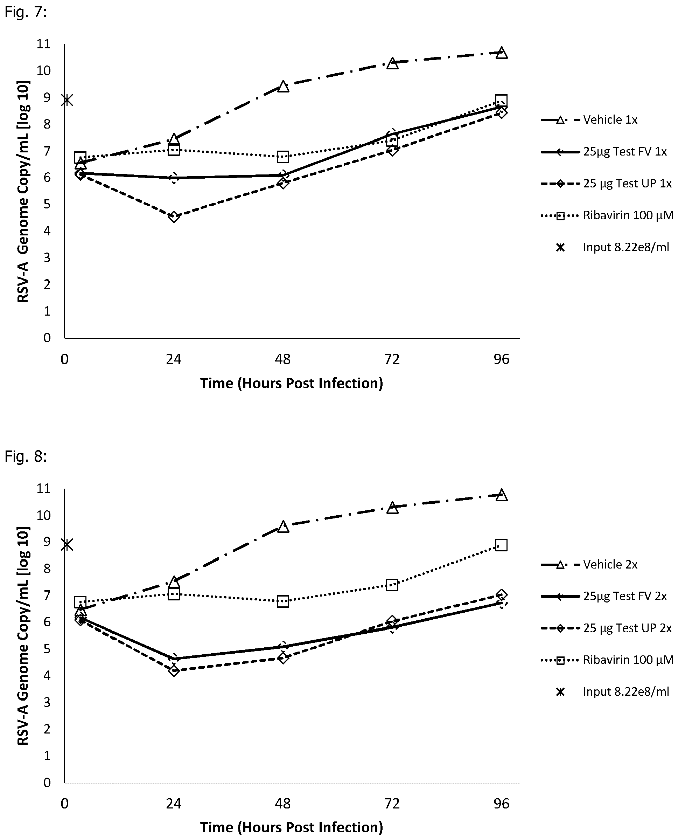

The inoculation concentration was higher than expected for RSV A, 10⁸/ml instead of 10⁶/ml. As expected, ribavirin decreased the viral load by 3 logs. As can be seen form Fig. 2 and 4, apical treatment with fucoidan compositions showed strong inhibitory effect on RSV A viral load in a dose dependent manner. Figure 8 shows that surprisingly, 25 µg Test FV AP and 25 µg Test UP AP even had more inhibitory effect than ribavirin. Apical treatment with the FV compositions (Fig. 2) resulted in a delay of viral replication up to 24 hours, wherein treatment with the UP compositions even resulted in a delay of viral replication up to 48 hours (Fig. 4). The results confirm that intranasal administration of the compositions of the invention is effective in reducing viral genome copy number of RSV A. The highest concentrated UP composition was most effective in reducing viral load. Apical administration to the nasal epithelia, e.g. in the form of a nasal spray, is a suitable form of administration.

Trans-epithelial electronic resistance (TEER) was measured at 24, 48, 72 and 96 hours. Treatment with Triton X-100, as expected, resulted in a harshly reduced TEER below 100 Ω.cm². Inserts that were not inoculated with virus but treated apically with fucoidan test compositions in the different concentrations (25 µg Test FV AP, 5 µg Test FV AP, 1 µg Test FV AP, 25 µg Test UP AP, 5 µg Test UP AP, 1 µg Test UP AP) showed TEER values in the normal range of the cell model, which confirms the results of Example 1. These concentrations of the compositions therefore are believed to be highly compatible with human nasal epithelia and to have no negative impact on tissue integrity. RSV-A infection induced an important increase of TEER, still in the normal range, from Day 3 in the non-treated control and in the vehicle. Treatment with basal application of the fucoidan compositions did not modify this RSV A induced deregulation. In contrast, treatment with apical application of the fucoidan compositions (for FV compositions with the 500 µg/ml attenuated TEER changes comparable to ribavirin basal treatment. In contrast, 5 µg Test FV AP, 25 µg Test FV AP and 25 µg Test UP AP apical attenuated TEER changes, comparable to treatment with positive antiviral control (ribavirin). Cytotoxicity was determined as in Example 1. Example 2 confirmed the results of Example 1: All cell culture inserts treated (except for the positive control) at all measured timepoints showed less than 5% cytotoxicity. RSV A thus also does not have cytotoxic effects on the cell model in the observed timeframe. This result shows that the compositions do not have cytotoxic effects and may be used *in vivo* as nasal spray compositions for humans without negative effects on the nasal epithelia.

The measurement of IL-8 levels on day 2 and day 4 showed that that, as expected, cytomix induced a high and significant increase in IL-8. Inserts that were not infected but treated apical fucoidan test compositions showed IL-8-levels comparable to those of the not infected, untreated inserts (Mock). This shows that the compositions do not provoke inflammatory response in nasal epithelia and are safe for use as a nasal spray in humans. RSV A in inoculated inserts, treated with vehicle, induced a significant increase of IL-8 release at both time points. This effect could be prevented by the positive control treatment with rupintrivir. Apical treatment with FV compositions very effectively reduced the RSV A induced IL-8 release in a dose dependent manner. Apical treatment with UP compositions was also effective in reducing IL-8 release on day 2, with the compositions with the highest concentration, 25 µg Test UP AP, also reduced IL-8 levels on day 4.

**Table 7: Overview of results of Example 2**

| Example 2 | | | | | | |
|---|---|---|---|---|---|---|
| | Virus induced deregulation | FV compositions | | UP compositions | | Positive antiviral control |
| | | BL | AP | BL | AP | |
| Viral genome copy number | | - | ↓ (3logs) | - | ↓ (>3logs) | ↓ (3logs) |
| TEER | ↑ | - | prevention | - | prevention | prevention |
| LDH | - | - | - | - | - | - |
| CBF | ↓ | ↑ | ↑ | - | ↑ | prevention |
| MCC | ↓ | - | prevention | - | ↑ | prevention |
| IL-8 | ↑ | ↓ | ↓ | - | ↓ | ↓ |
| IL-6 | ↑ | ↓ | ↓ | - | ↓ | ↑ |
| RANTES | ↑ | ↓ | ↓ | - | ↓ | ↓ |

| | | | | | | |
|---|---|---|---|---|---|---|
| ↑ : increase, ↓ : decrease, with the magnitude indicated in brackets, - : no effect | | | | | | |

### Example 3

### Experimental setup

The compositions used in Example 3 are given in Table 2. The experimental setup of Examples 3 and 4 is given in Table 8. T specifies the point in time with the number indicating the number of hours after the start of the experiment, e.g. T24h specifies the time point 24 hours after start of the experiment.

**Table 8: Experimental setup of Examples 3 and 4**

| Name of the test series | Number of repeats (inserts) | Viral infection | Treatment at | | | | | | | | |
|---|---|---|---|---|---|---|---|---|---|---|---|
| | | | T0h | T4h | T8h | T24h | T32h | T48h | T56h | T72h | T80h |
| Mock | 3 in each Example | no | / | / | / | / | / | / | / | / | / |
| 25µg Test FV or 25µg Test UP | 3 for each Test composition | no | 25 µg fucoidan | 25 µg fucoidan | / | 25 µg fucoidan | / | 25 µg fucoidan | / | 25 µg fucoidan | / |
| 25µg Test FV or 25µg Test UP | 3 for each Test composition | no | 25 µg fucoidan | 25 µg fucoidan | 25 µg fucoidan | 25 µg fucoidan | 25 µg fucoidan | 25 µg fucoidan | 25 µg fucoidan | 25 µg fucoidan | 25 µg fucoidan |
| Negative apical control once daily | 3 in each experiment | yes | 10 µl culture medium | 10 µl culture medium | / | 10 µl culture medium | / | 10 µl culture medium | / | 10 µl culture medium | / |
| Negative basal control once daily | 3 in each experiment | yes | 10 µl culture medium | 10 µl culture medium | / | 10 µl culture medium | / | 10 µl culture medium | / | 10 µl culture medium | / |
| Negative control twice daily | 3 in each experiment | yes | 10 µl culture medium | 10 µl culture medium | 10 µl culture medium | 10 µl culture medium | 10 µl culture medium | 10 µl culture medium | 10 µl culture medium | 10 µl culture medium | 10 µl culture medium |
| 25µg Test FV or 25µg Test UP | 3 for each Test composition | yes | 25 µg fucoidan | 25 µg fucoidan | / | 25 µg fucoidan | / | 25 µg fucoidan | / | 25 µg fucoidan | / |
| 25µg Test FV or 25µg Test UP | 3 for each Test composition | yes | 25 µg fucoidan | 25 µg fucoidan | 25 µg fucoidan | 25 µg fucoidan | 25 µg fucoidan | 25 µg fucoidan | 25 µg fucoidan | 25 µg fucoidan | 25 µg fucoidan |
| Positive (antiviral) control (5 µM rupintrivir or 100 µM ribavirin) | 3 in each experiment | yes | Added to the basal culture medium | / | / | Added to the basal culture medium | / | Added to the basal culture medium | / | Added to the basal culture medium | / |
| Positive control cytotoxicity | 3 in each experiment | yes | 50 µl of 10% Triton X-100 apically | / | / | 50 µl of 10% Triton X-100 apically | / | 50 µl of 10% Triton X-100 apically | / | 50 µl of 10% Triton X-100 apically | / |
| Positive control (inflammation, Cytomix) | 3 in each experiment | yes | | / | / | / | / | / | / | / | / |

### Protocol

At the start of the experiment, the inserts were washed with 200 µl culture medium for 10 minutes at 34°C and the inserts were transferred into a new culture plate containing 500 µl of culture medium per well. 10 µl of test composition (FV or UP) were pippeted onto the apical side of all inserts to be treated according to the experimental setup. All inserts were incubated for 1 hour.

At T1h, inserts to be occulated according to the experimental setup were inoculated as described above, with the respective virus (HRV-C15 in example 3, RSV in example 4). Some inserts were additionally treated with antiviral solution from the basal side as foreseen in the experimental setup. Inserts not to be inoculated according to the experimental setup ("Mock") were instead exposed to 100 µl of culture medium on the apical side. All inserts were incubated for 3 hours.

At T4h, all inserts were washed with three quick washing steps with 200 µl of culture medium at 34°C to remove the viral inoculum and unbound viruses. 200 µl of culture medium was added apically to all inserts and inserts were let sit for 20 minutes at 34°C. During this time, TEER of all inserts was measured. This apical liquid was then removed and was stored at -80°C until the virus copy number was determined. 10 µl of test composition (either 25 µg Test FV or 25 µg Test UP) were pipetted onto the apical side of all inserts to be treated according to the experimental setup. The inserts to be treated once daily according to the experimental setup were incubated for 20 hours. The inserts to be treated twice daily according to the experimental setup were incubated for 4 hours.

At T8h, 10 µl of test composition (either 25 µg Test FV or 25 µg Test UP) was pipetted onto the apical side of the inserts to be treated twice daily according to the experimental setup. These inserts were then incubated for 16 hours.

At T24h, 200 µl of culture medium was added apically to all inserts and inserts were let sit for 20 minutes at 34°C. During this time, TEER of all inserts was measured. This apical liquid was then removed and was stored at -80°C until the virus copy number was determined. 10 µl of test composition (either FV or UP) was pipetted onto the apical side of all inserts to be treated according to the experimental setup. The basal culture medium was removed and stored. All inserts were transferred to new culture plates containing 500 µl culture media per well. Antiviral solutions were added to the basal culture medium of the inserts to be treated with antiviral solution from the basal side as foreseen in the experimental setup. Inserts to be treated once daily according to the experimental setup were incubated for 24 hours. Inserts to be treated twice daily according to the experimental setup were incubated for 8 hours.

At T32h, 10 µl of test composition (either 25 µg Test FV or 25 µg Test UP) was pipetted onto the apical side of the inserts to be treated twice daily according to the experimental setup. These inserts were then incubated for 16 hours.

At T48h, 200 µl of culture medium was added apically to all inserts and inserts were let sit for 20 minutes at 34°C. During this time, TEER of all inserts was measured. This apical liquid was then removed and was stored at -80°C until the virus copy number was determined. The basal culture medium was removed and stored. This culture medium at 48hrs was later analysed for LDH, IL-8 and RANTES and the rest was stored. All inserts were transferred to new culture plates containing 500 µl culture media per well. Antiviral solutions were added to the basal culture medium of the inserts to be treated with antiviral solution from the basal side as foreseen in the experimental setup. 10 µl of test composition (either 25 µg Test FV or 25 µg Test UP) was pipetted onto the apical side of all inserts to be treated according to the experimental setup. Inserts to be treated once daily according to the experimental setup were incubated for 24 hours. Inserts to be treated twice daily according to the experimental setup were incubated for 8 hours.

At T56h, 10 µl of test composition (either 25 µg Test FV or 25 µg Test UP) was pipetted onto the apical side of the inserts to be treated twice daily according to the experimental setup. These inserts were then incubated for 16 hours.

At T72h, 200 µl of culture medium was added apically to all inserts and inserts were let sit for 20 minutes at 34°C. During this time, TEER of all inserts was measured. This apical liquid was then removed and was stored at -80°C until the virus copy number was determined. 10 µl of test composition (either 25 µg Test FV or 25 µg Test UP) was pipetted onto the apical side of all inserts to be treated according to the experimental setup. The basal culture medium was removed and stored. All inserts were transferred to new culture plates containing 500 µl culture media per well. Antiviral solutions were added to the basal culture medium of the inserts to be treated with antiviral solution from the basal side as foreseen in the experimental setup. Inserts to be treated once daily according to the experimental setup were incubated for 24 hours. Inserts to be treated twice daily were incubated for 8 hours.

At T80h, 10 µl of test composition (either 25 µg Test FV or 25 µg Test UP) was pipetted onto the apical side of the inserts to be treated twice daily according to the experimental setup. These inserts were then incubated for 16 hours.

At T96h, the ciliary beating frequency and the mucociliary clearance of all inserts were measured. 200 µl of culture medium was added apically to all inserts and inserts were let sit for 20 minutes at 34°C. During this time, TEER of all inserts was measured. This apical liquid was then removed and was stored at -80°C until the virus copy number was determined. The basal culture medium was removed and stored. This culture medium at 96hrs was later analysed for LDH, IL-8 and RANTES and the rest was stored.

### Results

### Reduction of viral genome copy number

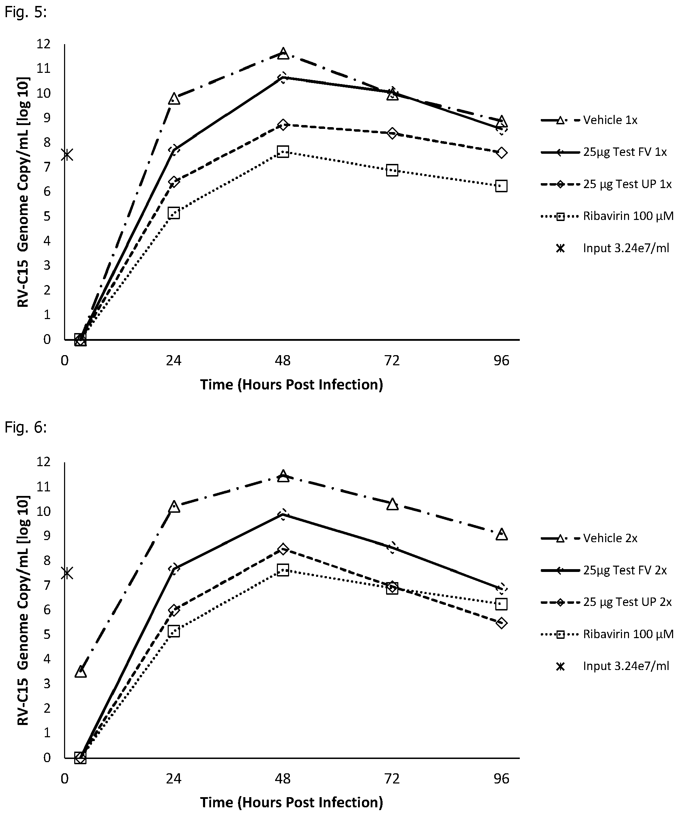

The HRV-C15 viral genome copy numbers of inserts treated with negative control, 25 µg Test FV once and twice daily, 25 µg Test UP once and twice daily and positive antiviral control are given Table 9 and are visualized in Figures 5 and 6. An overview of the results for all parameters is shown in Table 10.

**Table 9: Results of the measurement of the viral genome copy number in Example 3**

| | Mean Log 10 ((Copies/ml) + 1) | | | |
|---|---|---|---|---|
| Hours post infection | Vehicle 1x | 25µg Test FV 1x | 25 µg Test UP 1x | Ribavirin 100 µM |
| 3,5 | 0,00 | 0,00 | 0,00 | 0,00 |
| 24 | 9,82 | 7,70 | 6,41 | 5,14 |
| 48 | 11,64 | 10,65 | 8,73 | 7,63 |
| 72 | 9,99 | 10,06 | 8,37 | 6,89 |
| 96 | 8,88 | 8,54 | 7,59 | 6,24 |

| | Standard Deviation Log 10 ((Copies/ml) + 1) | | | |
|---|---|---|---|---|
| 3,5 | 0,00 | 0,00 | 0,00 | 0,00 |
| 24 | 9,91 | 7,62 | 6,63 | 5,33 |
| 48 | 10,71 | 10,74 | 8,40 | 7,43 |
| 72 | 10,04 | 9,83 | 8,12 | 6,72 |
| 96 | 8,60 | 7,42 | 7,34 | 6,08 |

| | Vehicle 2x | 25µg Test FV 2x | 25 µg Test UP 2x | Ribavirin 100 µM |
|---|---|---|---|---|
| 3,5 | 3,52 | 0,00 | 0,00 | 0,00 |
| 24 | 10,22 | 7,67 | 5,99 | 5,14 |
| 48 | 11,46 | 9,89 | 8,48 | 7,63 |
| 72 | 10,33 | 8,53 | 6,96 | 6,89 |
| 96 | 9,09 | 6,86 | 5,48 | 6,24 |

| | Standard Deviation Log 10 ((Copies/ml) + 1) | | | |
|---|---|---|---|---|
| 3,5 | 3,76 | 0,00 | 0,00 | 0,00 |
| 24 | 10,26 | 7,56 | 6,01 | 5,33 |
| 48 | 11,24 | 9,45 | 8,33 | 7,43 |
| 72 | 10,15 | 8,19 | 6,88 | 6,72 |
| 96 | 8,66 | 6,62 | 5,47 | 6,08 |

When the viral genome copy number was below the detection limit of the method, it was noted as 0.00 in the table and expressed as log 10 ((copies/ml) + 1) in the graphs. The number 1 was added to include values of 0.00 copies/ml in the graphs. As can be seen from table 9 and Figures 5 and 6, exposure to the compositions 25 µg Test VF and 25 µg Test UP significantly and consistently inhibited viral replication resulting in a reduced apical RV-C15 genome copy number, compared with vehicles, except for 25 µg Test VF once daily. Compositions comprising fucoidan have proved to be effective in reducing replication of RV-C15 in human nasal airway epithelia. Fucoidan compositions comprising fucoidan sourced from *Undaria pinnatifida* were more effective that compositions comprising fucoidan sourced from *Fucus vesiculosus.* Surprisingly, composition 25 µg Test UP even had, when applied twice daily, an effect that was comparable to that of antiviral positive control, (rupintrivir) (see e.g. Fig 6). Fucoidan compositions comprising fucoidan sourced from *Undaria pinnatifida* were effective in both, once daily and twice daily application scenario. The twice daily application of 25 µg Test UP resulted in a greater reduction of viral genome copies. The reduction in viral genome copy number was more pronounced with the twice daily application, for both, FV and UP compositions. The viral genome copy number was even reduced by more than three log scales at all time points by twice daily application of 25 µg Test UP. Basal rupintrivir exposure significantly reduced viral genome copy number and was thus a suitable positive control. Rupintrivir as the most effective inhibitor of RV-C15 replication.

Trans-epithelial electronic resistance (TEER) was measured at 24, 48, 72 and 96 hours. Treatment with Triton X-100 resulted in a harshly reduced TEER below 100 Ω.cm², and therefore was a suitable positive control agent for a negative impact on tissue integrity. Inserts that were not inoculated but treated with either 25 µg Test FV or 25 µg Test UP once or twice daily showed TEER values in the normal range of the cell model. Both compositions therefore are believed to be highly compatible with human nasal epithelia and to have no negative impact on tissue integrity. Inserts inoculated and treated with negative control (culture medium) showed significantly reduced TEER values at 24 hours post inoculation. TEER values subsequently recovered at 48, 72 and 96 hours to normal values. Inoculated inserts treated with 25 µg Test FV or 25 µg Test UP compositions once or twice daily also exhibited this transient TEER reduction. The drop in TEER occurred one or two days post infection. This is consistent with observations from previous studies that RV-C15 consistently induces transient loss of tissue integrity at day 1, 2 or 3 post infection, depending on the sensitivity of the tissue and/or the quantity of the virus inoculum. Inoculated inserts treated with positive control, apical rupintrivir, exhibited constantly high TEER values above 100 Ω.cm².

### Cytotoxicity

As per the measurement scheme used for cytotoxicity explained above, LDH levels of Triton X-100 treated inserts was set to be 100% cytotoxicity, and the LDH levels measured in all other inserts were put in relation to this value. All cell culture inserts treated (except for the positive control) at all measured timepoints showed less than 5% cytotoxicity. This result shows that the compositions do not have cytotoxic effects and may be used *in vivo* as nasal spray compositions for humans without negative effects on the nasal epithelia.

### Cilia beating frequency (CBF)

CBF of the Mock (not infected) inserts was about 6.5 Hz which is in the normal range for the cell model. All non-infected inserts also showed CBF between 5.3 to 7.3 Hz, which is also in the normal range for the cell model. All inserts infected with HRV-C15 showed a significantly reduced CBF to values of less than 1 Hz. Only inserts treated with positive control did not show this effect and still showed CBF of about 6 Hz.

### Mucociliary clearance (MCC)

Mock showed a bead velocity of 50 µm/s at 34°C. Similar to the results in CBF, MCC in the non-infected inserts was similar to mock and in the normal range of about 40 µm/s to 90 µm/s. However, inserts infected with HRV-C15 showed significant reduction of MCC with values below 10 µm/s, irrespective of the treatment, with the exception of treatment with positive control which prevented the decrease in MCC.

### Interleukin 8

IL-8 levels in the basal media were measured on day 2 and day 4. Inserts not inoculated and not treated inserts (mock) showed a basal concentration of IL-8 of about 20 ng/ml on day 2 and 4. Cytomix induced a high and significant increase in IL-8 in non-inoculated inserts and therefore was an effective positive control agent for induction of inflammatory response. Treatment of the not-inoculated inserts with 25 µg Test FV or 25 µg Test UP compositions once or twice daily resulted in consistently low IL-8-levels of about 10 ng/ml to about 30 ng/ml. RV-C15 in inoculated inserts, treated with vehicle, induced a significant increase of IL-8 release compared to mock. FV and UP compositions could not modify the RV-C15 induced IL-8 release at day 2 compared to mock. However, twice daily treatment with FV or UP compositions prevented RV-C15 induced IL-8 release at day 4. Furthermore, in inoculated inserts treated with FV or UP compositions, IL-8 levels were reduced significantly compared to their respective control (inserts treated once or twice daily with vehicle), but no statistically significant changes at day 4.

### RANTES

The level of RANTES was determined in the basal media on day 2 and day 4. Cytomix induced a high and significant increase in RANTES in non-inoculated inserts and therefore was an effective positive control agent. Infection with RV-C15 induced significant increase of RANTES release in the cell model. Rupintrivir effectively prevented the virus-induced increase of RANTES release. The compositions 25 µg Test FV or 25 µg Test UP could not modify the RV-C15-induced RANTES release at day 2 in a statistically significant manner. However, statistically significant reduction of RANTES release was observed for the twice daily application of 25 µg Test FV or 25 µg Test UP compositions on day 4. Statistical comparison of 25 µg Test FV or 25 µg Test UP UP compositions to their respective control (vehicle once or twice daily) showed significant decrease of RV-C15 induced RANTES release at Day 2 for 25 µg Test FV once and twice daily and for 25 µg Test UP twice daily.

**Table 10: Overview of results of Example 3**

| Example 3 | | | | | | |
|---|---|---|---|---|---|---|
| | Virus induced deregulation | FV compositions | | UP compositions | | Positive antiviral control |
| | | 1x | 2x | 1x | 2x | |
| Viral genome copy number | | ↓ (1-2logs) | ↓ (2-3logs) | ↓ (2-3logs) | ↓ (3-4logs) | ↓ (2-5logs) |
| TEER | ↓ | - | - | - | - | prevention |
| LDH | - | - | - | - | - | - |
| CBF | ↓ | - | - | - | - | prevention |
| MCC | ↓ | - | - | - | - | prevention |
| IL-8 | ↑ | ↓ (day 4) | ↓ (day 4) | ↓ (day 4) | ↓ (day 4) | prevention |
| RANTES | ↑ | - | ↓ (day 4) | - | ↓ (day 4) | prevention |

| | | | | | | |
|---|---|---|---|---|---|---|
| ↑ : increase, ↓ : decrease, with the magnitude indicated in brackets, - : no effect | | | | | | |

### Example 4

The compositions used in Example 4 are indicated in Table 2. The experimental setup and the protocol of Example 4 were the same as for Example 3 and are given in Table 9, except that respiratory syncytial virus A was used as viral inoculum, and consequently ribavirin was used as positive antiviral control.

### Results

The RSV A genome copy number of the inserts treated with negative control, 25 µg Test FV or 25 µg Test UP once or twice daily and positive antiviral control inserts are given in Table 11 and are visualised in Figures 7 and 8. An overview of the results for all parameters is shown in Table 12.

**Table 11: Results of the measurement of the viral genome copy number in Example 4**

| | Mean Log 10 ((Copies/ml) + 1) | | | |
|---|---|---|---|---|
| Hours post infection | Vehicle 1x | 25µg Test FV 1x | 25 µg Test UP 1x | Ribavirin 100 µM |
| 3,5 | 6,57 | 6,18 | 6,12 | 6,76 |
| 24 | 7,45 | 6,00 | 4,55 | 7,06 |
| 48 | 9,45 | 6,09 | 5,80 | 6,79 |
| 72 | 10,30 | 7,64 | 7,04 | 7,40 |
| 96 | 10,70 | 8,65 | 8,43 | 8,89 |

| | Standard Deviation Log 10 ((Copies/ml) + 1) | | | |
|---|---|---|---|---|
| 3,5 | 6,38 | 6,08 | 5,95 | 6,78 |
| 24 | 7,24 | 6,20 | 4,71 | 6,97 |
| 48 | 8,97 | 6,02 | 5,45 | 6,37 |
| 72 | 9,79 | 7,39 | 6,78 | 7,04 |
| 96 | 10,54 | 8,73 | 8,39 | 8,86 |

| | Mean Log 10 ((Copies/ml) + 1) | | | |
|---|---|---|---|---|
| | Vehicle 1x | 25µg Test FV 1x | 25 µg Test UP 1x | Ribavirin 100 µM |
| 3,5 | 6,48 | 6,18 | 6,08 | 6,76 |
| 24 | 7,54 | 4,64 | 4,20 | 7,06 |
| 48 | 9,61 | 5,10 | 4,67 | 6,79 |
| 72 | 10,32 | 5,82 | 6,05 | 7,40 |
| 96 | 10,79 | 6,74 | 7,04 | 8,89 |

| | Standard Deviation Log 10 ((Copies/ml) + 1) | | | |
|---|---|---|---|---|
| 3,5 | 6,05 | 5,90 | 4,60 | 6,78 |
| 24 | 7,38 | 4,64 | 4,43 | 6,97 |
| 48 | 9,04 | 4,35 | 4,74 | 6,37 |
| 72 | 9,28 | 5,16 | 6,20 | 7,04 |
| 96 | 10,69 | 6,80 | 7,17 | 8,86 |

When the viral genome copy number was below the detection limit of the method, it was noted as 0.00, and was also represented in the graphs as 0.
As can be seen from Table 12 and Figures 7 and 8, exposure to the FV or UP compositions significantly inhibited viral replication resulting in a reduced apical RSV A genome copy number, compared with vehicles. The reduction in viral genome copy number was more pronounced already at 24 hours with the twice daily application, for both compositions, compared to once daily. Significantly, the twice daily application of the 25 µg Test UP composition even resulted in a viral load lower than that observed in ribavirin treated inserts (Fig. 8)
Compositions comprising fucoidan have proved to be effective in reducing replication of RSV A in human nasal airway epithelia. Compositions comprising fucoidan derived from *Fucus vesiculosus* or *Undaria pinnatifida* should similar results in RSV A replication inhibition. Both compositions were effective in the once daily and the twice daily application scenario, whilst inhibition was higher with twice daily dosing.

### TEER

Trans-epithelial electronic resistance (TEER) was measured at 24, 48, 72 and 96 hours. Treatment with Triton X-100 resulted in a harshly reduced TEER below 100 Ω.cm², and therefore was a suitable positive control agent for a negative impact on tissue integrity. Inserts that were not inoculated but treated with either 25 µg Test FV or 25 µg Test UP once or twice daily showed TEER values in the normal range of the cell model. Both compositions therefore are believed to be highly compatible with human nasal epithelia and to have no negative impact on tissue integrity. Inoculated inserts treated with vehicle (culture media), 25 µg Test FV or 25 µg Test UP compositions once or twice daily or basal ribavirin exhibited TEER values similar to that of the mock inserts, i.e. in the normal range. RSV A infection thus does not have the same effect on TEER as infection with HRV-C15 described above.

### Cytotoxicity

As per the measurement scheme used for cytotoxicity explained above, LDH levels of Triton X-100 treated inserts was set to be 100% cytotoxicity, and the LDH levels measured in all other inserts were put in relation to this value. The inserts not inoculated but treated with the FV or UP compositions once or twice daily, the inserts inoculated and treated with vehicle, FV or UP once or twice daily or rupintrivir did show a cytotoxicity below 5%.
These results show that compositions comprising fucoidan do not have cytotoxic effects and may be used *in vivo* as nasal spray compositions for humans without negative effects on the nasal epithelia.

### Cilia beating frequency

CBF of the Mock (not infected) inserts was about 6 Hz which is in the normal range for the cell model. All inserts that were not inoculated, irrespective of their treatment, showed cilia beating frequencies between 4.5 Hz to 7.2 Hz at room temperature, which are in the normal range for the cell model. RSV A infected inserts treated with negative control only showed a significant decrease of the CBF on day 4 to below 1 Hz. This RSV A induced cilia impairment was prevented by all interventions, i.e. once or twice daily treatment of the inoculated inserts with 25 µg Test FV or 25 µg Test UP compositions or ribavirin. The compositions are therefore effective in prevention of RSV A induced ciliary beating impairment.

### Mucociliary clearance (MCC)

Mock showed a bead velocity of 90 µm/s at 34°C. The inserts that were not inoculated (treated or untreated) all exhibited comparable, high MCC values, except for once daily treatment with UP compositions which resulted in a small significant decrease of MCC. RSV A infected inserts treated with negative control only showed a significant decrease of the MCC on day 4 to below 10 µm/s. Treatment with 25 µg Test FV or 25 µg Test UP compositions once or twice daily or basal ribavirin attenuated this RSV A induced MCC reduction but could not restore the MCC to the normal range. Only twice daily treatment with 25 µg Test FV resulted in a MCC value of about 50 µm/s which is in the normal range. Twice daily treatment with 25 µg Test FV therefore was effective in preventing RSV A induced MCC impairment.

### Interleukin 8

IL-8 levels in the basal media were measured on day 2 and day 4. Inserts not inoculated and not treated inserts (mock) showed a basal concentration of IL-8 of about 20 ng/ml on day 2 and 4. Cytomix induced a high and significant increase in IL-8 in non-inoculated inserts, compared to mock, and therefore was an effective positive control agent for induction of inflammatory response. Treatment of the not-inoculated inserts with 25 µg Test FV or 25 µg Test UP once or twice daily resulted in consistently low IL-8-levels of about 10 ng/ml to about 40 ng/ml. RSV A-inoculated inserts, treated with vehicle, showed a significant increase of IL-8 release compared to mock, with the IL-8 level higher on day 4 than on day 2. Treatment with 25 µg Test FV or 25 µg Test UP once or twice daily or basal ribavirin prevented the RSV A induced IL-8 release, except for 25 µg Test UP once daily treatment. Furthermore, in inoculated inserts treated with FV or UP compositions once or twice daily, IL-8 levels were reduced significantly on day 2 and day 4 compared to their respective control (inserts treated once or twice daily with vehicle).

### RANTES

The level of RANTES was determined in the basal media on day 2 and day 4. Cytomix induced a high and significant increase in RANTES in non-inoculated inserts and therefore was an effective positive control agent. Infection with RSV A induced significant increase of RANTES release in the cell model which was higher on day 4 than on day 2. Ribavirin effectively prevented the virus-induced increase of RANTES release. Both test compositions, 25 µg Test FV and 25 µg Test UP also prevented the RSV A-induced RANTES release, except for 25 µg Test UP once daily. For both test compositions, the RANTES level was lower in the twice daily dosing scenario than in the once daily dosing scenario. Statistical comparison of 25 µg Test FV or 25 µg Test UP to their respective control (negative control once or twice daily) showed significant decrease of RSV A induced RANTES release at day 4 in the once daily dosing and at both days in the twice daily dosing. The test compositions thus are effective in preventing the RSV A-induced RANTES release, wherein twice daily dosing is more effective and effective already at day 2.

**Table 12: Overview of results of Example 4**

| Example 4 | | | | | | |
|---|---|---|---|---|---|---|
| | Virus induced deregulation | FV compositions | | UP compositions | | Positive antiviral control |
| | | 1x | 2x | 1x | 2x | |
| Viral genome copy number | | ↓ (1-3logs) | ↓ (3-4logs) | ↓ (2-3logs) | ↓ (3-4logs) | ↓ (0-3logs) |
| TEER | - | - | - | - | - | - |
| LDH | - | - | - | - | - | - |
| CBF | ↓ | prevention | prevention | prevention | prevention | prevention |
| MCC | ↓ | attenuation | prevention | attenuation | attenuation | attenuation |
| IL-8 | ↑ | prevention | prevention | - | prevention | prevention |
| RANTES | ↑ | prevention | prevention | - | prevention | prevention |

| | | | | | | |
|---|---|---|---|---|---|---|
| ↑ : increase, ↓ : decrease, with the magnitude indicated in brackets, - : no effect | | | | | | |

### Example 5

### Experimental setup

The compositions used in Example 5 are given in Table 3. Each intervention was performed in three different inserts (i.e. three replicates) at T0h, T4h, T8h, T24h, T32h, T48h, T56h, T72h and T80h. Three inserts each were treated apically with the fucoidan test compositions in the different concentrations. Three inserts were treated with the Boots dual defense product by pipetting of 10 µl of the product onto the apical side of the cell culture. Negative controls were: Untreated and uninfected inserts (Mock), uninfected inserts treated with the Boots dual defense product as the test inserts, uninfected inserts each treated with either 25 µg Test UP or 200 µg Test UP and three infected inserts treated apically with cell culture medium. Positive controls were: infected inserts treated with rupintrivir basal and apical cell culture medium, inserts treated with Triton X-100 and inserts treated with Cytomix.

The protocol was as in Example 3, but with the compositions and interventions as described above in the experimental setup for Example 5.

### Results

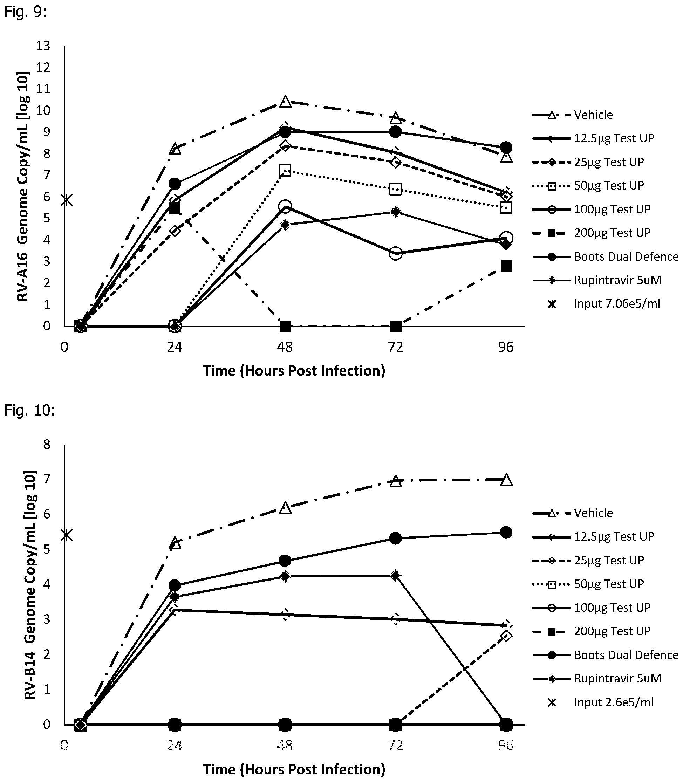

The HRV-A16 genome copy number of the inserts treated with negative control, 12.5µg Test UP, 25 µg Test UP, 50µg Test UP, 100µg Test UP, 200µg Test UP twice daily and positive antiviral control inserts are given in Table 13 and are visualised in Figure 9.

**Table 13: Results of the measurement of the viral genome copy number in Example 5**

| | Mean Log 10 ((Copies/ml) + 1) | | | | | | | |
|---|---|---|---|---|---|---|---|---|
| Hours post infection | Vehicle | 12.5µg Test UP | 25µg Test UP | 50µg Test UP | 100µg Test UP | 200µg Test UP | Boots Dual Defence | Rupintrivir 5µM |
| 3,5 | 0,00 | 0,00 | 0,00 | 0,00 | 0,00 | 0,00 | 0,00 | 0,00 |
| 24 | 8,25 | 5,85 | 4,43 | 0,00 | 0,00 | 5,49 | 6,59 | 0,00 |
| 48 | 10,44 | 9,21 | 8,37 | 7,21 | 5,54 | 0,00 | 8,99 | 4,70 |
| 72 | 9,68 | 8,05 | 7,61 | 6,35 | 3,37 | 0,00 | 9,02 | 5,30 |
| 96 | 7,89 | 6,21 | 6,01 | 5,49 | 4,09 | 2,81 | 8,28 | 3,77 |

| | Standard Deviation Log 10 ((Copies/ml) + 1) | | | | | | | |
|---|---|---|---|---|---|---|---|---|
| 3,5 | 0 | 0 | 0 | 0 | 0 | 0 | 0 | 0 |
| 24 | 8,00 | 5,75 | 4,41 | 0,00 | 0,00 | 5,73 | 6,43 | 0,00 |
| 48 | 10,26 | 9,09 | 7,93 | 7,27 | 5,73 | 0,00 | 8,80 | 4,64 |
| 72 | 9,73 | 8,06 | 7,61 | 6,44 | 3,40 | 0,00 | 9,04 | 5,24 |
| 96 | 7,73 | 6,21 | 6,13 | 4,55 | 4,31 | 3,05 | 8,15 | 4,01 |

The inoculation concentration was 7.1x10⁵/ml in Example 5. Treatment with the fucoidan compositions, at all concentrations, reduced apical RV-A16 genome copy number in the cell model and the difference was significant at 48 hours. Comparing efficacy of the fucoidan compositions to positive antiviral control rupintrivir, the concentration of 10 mg/ml seemed to be close to the effect of rupintrivir. The composition with the highest concentration of fucoidan of 20 mg/ml was able to completely block RV-A16 replication at Day 2 and 3, but some inserts showed replication at Day 1 and 4 (D1: n=1, while undetectable virus for another two inserts of the triplicate, D4: n=1 insert, not the same, while undetectable virus for another two inserts of the triplicate).
In the TEER test, it was observed that all fucoidan compositions at all concentrations preserved the tissue integrity in the non-infected inserts. Also, no cytotoxicity was observed in the LDH-measurements. The tested fucoidan compositions are thus compatible with human nasal epithelia.

### Example 6

### Experimental setup

The compositions and experimental setup and protocol in Example 6 were the same as in Example 5, except that HRV-B14 was used as viral inoculum.

### Results

The HRV-B14 genome copy number of the inserts treated with negative control, 12.5µg Test UP, 25 µg Test UP, 50µg Test UP, 100µg Test UP, 200µg Test UP twice daily and positive antiviral control inserts are given in Table 14 and are visualised in Figure 10.

**Table 14: Results of the measurement of the viral genome copy number in Example 6**

| | Mean Log 10 ((Copies/ml) + 1) | | | | | | | |
|---|---|---|---|---|---|---|---|---|
| | Vehicle | 12.5µg Test UP | 25µg Test UP | 50µg Test UP | 100µg Test UP | 200µg Test UP | Boots Dual Defence | Rupintrivir 5µM |
| 3,5 | 0,00 | 0,00 | 0,00 | 0,00 | 0,00 | 0,00 | 0,00 | 0,00 |
| 24 | 5,21 | 3,27 | 0,00 | 0,00 | 0,00 | 0,00 | 3,97 | 3,65 |
| 48 | 6,21 | 3,14 | 0,00 | 0,00 | 0,00 | 0,00 | 4,67 | 4,23 |
| 72 | 6,97 | 3,01 | 0,00 | 0,00 | 0,00 | 0,00 | 5,32 | 4,26 |
| 96 | 7,00 | 2,83 | 2,54 | 0,00 | 0,00 | 0,00 | 5,49 | 0,00 |

| | Standard Deviation Log 10 ((Copies/ml) + 1) | | | | | | | |
|---|---|---|---|---|---|---|---|---|
| 3,5 | 0,00 | 0,00 | 0,00 | 0,00 | 0,00 | 0,00 | 0,00 | 0,00 |
| 24 | 4,81 | 3,31 | 0,00 | 0,00 | 0,00 | 0,00 | 3,91 | 3,78 |
| 48 | 5,86 | 3,16 | 0,00 | 0,00 | 0,00 | 0,00 | 4,73 | 4,47 |
| 72 | 6,81 | 3,25 | 0,00 | 0,00 | 0,00 | 0,00 | 5,44 | 4,49 |
| 96 | 7,14 | 3,07 | 2,78 | 0,00 | 0,00 | 0,00 | 5,27 | 0,00 |

The RV-B14 inoculation concentration was 2.6x10⁵/ml. Exposure to the fucoidan compositions, at all concentrations, dramatically reduced apical RV-B14 genome copy number in the cell model. Virus could be detected only in the inserts treated with 12.5µg Test UP, and in inserts treated with 25µg Test UP on Day 4. Comparing effect of the fucoidan compositions to positive antiviral control rupintrivir, all concentrations of fucoidan compositions were more effective than rupintrivir until Day 3. At Day 4 no more virus was detected with rupintrivir, but virus was detected in inserts treated with 12.5µg Test UP and 25µg Test UP. In the TEER test, it was observed that all fucoidan compositions at all concentrations preserved the tissue integrity in the non-infected inserts. Also, no cytotoxicity was observed in the LDH-measurements. The tested fucoidan compositions are thus compatible with human nasal epithelia.

## Claims

1. Pharmaceutical composition comprising fucoidan for use in a method for the treatment or the prevention, by intranasal administration, of an infection with rhinovirus or respiratory syncytial virus in a human.

2. Pharmaceutical composition for use according to claim 1 wherein the infection is HRV-C15 infection, HRV-A16 infection or HRV-B14 infection.

3. Pharmaceutical composition for use according to claim 1 wherein the infection is respiratory syncytial virus type A infection.

4. Pharmaceutical composition for use according to any of the preceding claims wherein the pharmaceutical compositions is a nasal spray.

5. Pharmaceutical composition for use according to any of the preceding claims wherein the pharmaceutical composition comprises 0.1% to 2.7% (w/w) fucoidan

6. Pharmaceutical composition for use according to any of the preceding claims wherein the pharmaceutical composition is an aqueous solution.

7. Pharmaceutical composition according to claim 6 wherein the pharmaceutical composition comprises a sodium chloride solution.

8. Pharmaceutical composition for use according to any of the preceding claims wherein the sodium chloride solution is isotonic.

9. Pharmaceutical composition for use according to any of the preceding claims wherein the pharmaceutical composition is administered intranasally two or three times daily into each nostril of a subject.

10. Pharmaceutical composition for use according to any of the preceding claims wherein the pharmaceutical composition is administered in a dose of 4 to 8 mg fucoidan into each nostril of a subject per administration.

11. Pharmaceutical composition for use according to any of the preceding claims, wherein the fucoidan is fucoidan extracted from Undaria pinnatifida.

12. Pharmaceutical composition for use according to any of the preceding claims, wherein the fucoidan has an average molecular weight of 150 kDa and a lower molecular weight cut-off of 10 kDa.

13. Pharmaceutical composition for use according to any of the preceding claims, wherein the fucoidan has a sulfate content of 20% to 40% (w/w).

14. Pharmaceutical composition according to any of the preceeding claims for use in a method for prevention of spread of rhinovirus or respiratory syncytial virus in a human population, wherein subjects are tested for infection with rhinovirus or respiratory syncytial virus and infected subjects are treated with intranasal administration of the pharmaceutical composition
